(19) Europäisches Patentamt / European Patent Office / Office européen des brevets

(11) **EP 4 401 008 A1**

(12) **EUROPEAN PATENT APPLICATION**
published in accordance with Art. 153(4) EPC

(43) Date of publication:
**17.07.2024 Bulletin 2024/29**

(51) International Patent Classification (IPC):
**G06N 3/04** (2023.01)

(21) Application number: **23867179.6**

(86) International application number:
**PCT/CN2023/113188**

(22) Date of filing: **15.08.2023**

(87) International publication number:
**WO 2024/060886 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.09.2022 CN 202211168243**

(71) Applicant: **Tencent Technology (Shenzhen) Company Limited**
**Shenzhen, Guangdong, 518057 (CN)**

(72) Inventor: **XU, Shaoyong**
**Shenzhen, Guangdong 518057 (CN)**

(74) Representative: **Gevers Patents**
**De Kleetlaan 7A**
**1831 Diegem (BE)**

(54) **METHOD FOR TRAINING BINDING AFFINITY MEASUREMENT MODEL, AND BINDING AFFINITY MEASUREMENT METHOD**

(57) Provided are a method for training a binding affinity measurement model, and a binding affinity measurement method, which belong to the technical field of biology. The method comprises: acquiring protein structure data of a sample protein, small-molecule structure data of a sample drug small-molecule, and a sample labeling result (201); calling a neural network model to determine structural data of a plurality of target complex conformations on the basis of the protein structure data and the small-molecule structure data (202); calling the neural network model to determine a sample prediction result on the basis of the structural data of each target complex conformation (203); and training the neural network model by using the sample prediction result and the sample labeling result, to obtain a binding affinity measurement model (204).

Obtain protein structure data of a sample protein, small molecule structure data of a sample small molecule, and a sample labeling data — 201

Determine structure data of a plurality of conformational ensembles by inputting the protein structure data and the small molecule structure data into an initial predictive neural network model — 202

Determine a predictive binding affinity between the sample protein and the sample small molecule by inputting the structure data of the conformational ensembles into the initial predictive neural network model — 203

Train the initial predictive neural network model using the sample prediction result and the sample labeling data to obtain the binding affinity detection model — 204

FIG. 2

**Description**

RELATED APPLICATION

This application claims priority to Chinese Patent Application No. 202211168243.7, entitled "METHOD FOR TRAINING BINDING AFFINITY DETECTION MODEL AND BINDING AFFINITY DETECTION METHOD" and filed on September 23, 2022.

FIELD OF THE TECHNOLOGY

Embodiments of the present disclosure relate to the field of biotechnologies, and specifically, to a method for obtaining a binding affinity detection model and a binding affinity detection method.

BACKGROUND OF THE DISCLOSURE

In the field of biotechnologies, proteins participate in life activities such as catalysis, immunity, and metabolism, and are important substances that form organisms. Generally, a binding affinity between a protein and a small drug molecule may be measured, and by analyzing the binding affinity, a druggability potential of the small drug molecule may be obtained.

In the related art, a binding affinity detection model may be used to detect the binding affinity between the protein and the small drug molecule. Since the binding affinity between the protein and the small drug molecule is closely related to the druggability potential of the small drug molecule, how to train a binding affinity detection model with higher accuracy becomes an important technology.

SUMMARY

The present disclosure provides a method for obtaining a binding affinity detection model for detecting a binding affinity between a given protein and a given small molecule, which may be configured for improving accuracy of the binding affinity detection model. The technical solutions include the following content.

According to an aspect, a method for obtaining a binding affinity detection model for detecting a binding affinity between a given protein and a given small molecule is provided, performed by an electronic device, and the method including:

obtaining protein structure data of a sample protein, small molecule structure data of a sample small molecule, and sample labeling data, the sample labeling data representing a binding affinity between the sample protein and the sample small molecule;

determining structure data of a plurality of conformational ensembles by inputting the protein structure data and the small molecule structure data into an initial predictive neural network model, the plurality of conformational ensembles each indicating a predictive binding structure between the sample protein and the sample small molecule;

determining a predictive binding affinity between the sample protein and the sample small molecule by inputting the structure data of the conformational ensembles into the initial predictive neural network model; and

training the initial predictive neural network model using the sample prediction result and the sample labeling data to obtain the binding affinity detection model.

According to another aspect, a binding affinity detection method is provided, performed by an electronic device, and the method including:

obtaining structure data of a given protein, structure data of a given small molecule, and a binding affinity detection model, the binding affinity detection model being obtained through training by using the method for obtaining a binding affinity detection model described above;

determining structure data of a plurality of reference conformational ensembles by inputting the structure data of the given protein and the structure data of the given small molecule into the binding affinity detection model, the plurality of reference conformational ensembles each indicating a structure obtained through prediction and formed by binding the given protein and the given small molecule; and

determining a binding affinity detection result between the given protein and the given small molecule by inputting the structure data of the reference conformational ensembles into the binding affinity detection model.

According to another aspect, an apparatus for obtaining a binding affinity detection model for detecting a binding affinity between a given protein and a given small molecule is provided, including:

an obtaining module, configured to obtain protein structure data of a sample protein, small molecule structure data of a sample small molecule, and sample labeling data, the sample labeling data representing a binding affinity between the sample protein and the sample small molecule;

a determining module, configured to determine structure data of a plurality of conformational ensembles by inputting the protein structure data and the small molecule structure data into an initial predictive neural network model, the plurality of conformational ensembles each indicating a predictive binding structure between the sample protein and the sample small molecule;

the determining module, further configured to determine a predictive binding affinity between the sample protein and the sample small molecule by inputting the structure data of the conformational ensembles into the initial predictive neural network model; and

a training module, configured to train the initial predictive neural network model using the sample prediction result and the sample labeling data to obtain the binding affinity detection model.

According to another aspect, a binding affinity detection apparatus is provided, including:

an obtaining module, configured to obtain structure data of a given protein, structure data of a given small molecule, and a binding affinity detection model, the binding affinity detection model being obtained through training by using the method for obtaining a binding affinity detection model described above;

a determining module, configured to determine structure data of a plurality of reference conformational ensembles by inputting the structure data of the given protein and the structure data of the given small molecule into the binding affinity detection model, the plurality of reference conformational ensembles each indicating a structure obtained through prediction and formed by binding the given protein and the given small molecule; and

the determining module, further configured to determine a binding affinity detection result between the given protein and the given small molecule by inputting the structure data of the reference conformational ensembles into the binding affinity detection model.

According to another aspect, an electronic device is provided. The electronic device includes a processor and a memory, the memory stores at least one computer program, and the at least one computer program is loaded and executed by the processor to cause the electronic device to implement the foregoing method for obtaining a binding affinity detection model, or implement the foregoing binding affinity detection method.
According to another aspect, a non-transitory computer-readable storage medium is further provided. The non-transitory computer-readable storage medium stores at least one computer program, and the at least one computer program is loaded and executed by a processor to cause an electronic device to implement the foregoing method for obtaining a binding affinity detection model, or implement the foregoing binding affinity detection method.
According to another aspect, a computer program or a computer program product is further provided. The computer program or the computer program product stores at least one computer program, and the at least one computer program is loaded and executed by a processor to cause an electronic device to implement the foregoing method for obtaining a binding affinity detection model, or implement the foregoing binding affinity detection method.
In the technical solutions provided in the present disclosure, any conformational ensemble is a structure obtained through prediction and formed by binding of a sample protein and a sample small molecule. Through a plurality of conformational ensembles, a probability of covering a real structure formed by the binding of the sample protein and the sample small molecule may be improved. That is, the plurality of conformational ensembles can more accurately express the real structure formed by the binding of the sample protein and the sample small molecule, so that a sample prediction result is more accurate when a neural network model is called to determine the sample prediction result based on structure data of the conformational ensembles. In this way, when the neural network model is trained by using the sample prediction result to obtain a binding affinity detection model, the accuracy of the binding affinity detection model can be improved, thereby improving the accuracy of a binding affinity detection result.

BRIEF DESCRIPTION OF THE DRAWINGS

FIG. 1 is a schematic diagram of an implementation environment of a method for obtaining a binding affinity detection model or a binding affinity detection method according to an embodiment of the present disclosure.

FIG. 2 is a flowchart of a method for obtaining a binding affinity detection model according to an embodiment of the present disclosure.

FIG. 3 is a schematic diagram of a spatial structure and a graph structure of a candidate conformational ensemble according to an embodiment of the present disclosure.

FIG. 4 is a schematic diagram of extracting a conformational feature of a candidate conformational ensemble according to an embodiment of the present disclosure.

FIG. 5 is a schematic diagram of determining a weighted fusion feature according to an embodiment of the present disclosure.

FIG. 6 is a schematic diagram of determining a sample prediction result according to an embodiment of the present disclosure.

FIG. 7 is a schematic diagram of obtaining a binding affinity detection model according to an embodiment of the present disclosure.

FIG. 8 is a flowchart of a binding affinity detection method according to an embodiment of the present disclosure.

FIG. 9 is a schematic diagram of a binding affinity detection process according to an embodiment of the present disclosure.

FIG. 10 is a schematic diagram of comparison of test indicators according to an embodiment of the present disclosure.

FIG. 11 is a schematic structural diagram of an apparatus for obtaining a binding affinity detection model according to an embodiment of the present disclosure.

FIG. 12 is a schematic structural diagram of a binding affinity detection apparatus according to an embodiment of the present disclosure.

FIG. 13 is a schematic structural diagram of a terminal device according to an embodiment of the present disclosure.

FIG. 14 is a schematic structural diagram of a server according to an embodiment of the present disclosure.

DESCRIPTION OF EMBODIMENTS

To make objectives, technical solutions, and advantages of the present disclosure clearer, implementations of the present disclosure are further described below in detail with reference to the accompanying drawings.

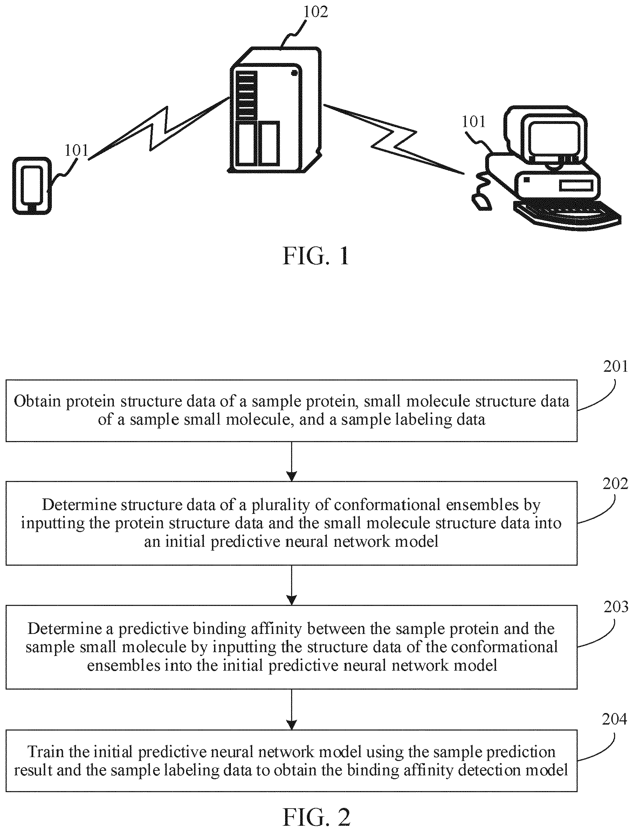

FIG. 1 is a schematic diagram of an implementation environment of a method for obtaining a binding affinity detection model for detecting a binding affinity between a given protein and a given small molecule or a binding affinity detection method according to an embodiment of the present disclosure. As shown in FIG. 1, the implementation environment includes a terminal device 101 and a server 102. In the embodiments of the present disclosure including the embodiments of both the claims and specification (hereinafter referred to as "all embodiments of the present disclosure"), the method for obtaining a binding affinity detection model or the binding affinity detection method may be performed by the terminal device 101, or may be performed by the server 102, or may be performed by the terminal device 101 and the server 102 together.

The terminal device 101 may be a smartphone, a game console, a desktop computer, a tablet computer, a laptop portable computer, a smart television, a smart in-vehicle device, a smart speech interaction device, or a smart household appliance. The server 102 may be one server, a server cluster including a plurality of servers, or any one of a cloud computing platform and a virtualization center. This is not limited in this embodiment of the present disclosure.

The server 102 is communicatively connected to the terminal device 101 through a wired network or a wireless network. The server 102 may have functions such as data processing, data storage, and data sending and receiving. This is not limited in this embodiment of the present disclosure. A quantity of the terminal devices 101 and a quantity of the servers 102 are not limited, and there may be one or more terminal devices 101 and servers 102.

The method for obtaining a binding affinity detection model and the binding affinity detection method provided in all embodiments of the present disclosure may be implemented based on an artificial intelligence (AI) technology. The AI technology is a theory, method, technology and application system that uses a digital computer or a machine controlled by the digital computer to simulate, extend and expand human intelligence, perceive an environment, acquire knowledge, and use the knowledge to obtain an optimal result. In other words, AI is a comprehensive technology in computer science and attempts to understand the essence of intelligence and produce a new intelligent machine that can react in a manner similar to human intelligence. The AI is to study design principles and implementation methods of various intelligent machines, to enable a machine to have functions of perception, reasoning, and decision-making.

The AI technology is a comprehensive discipline, relating to a wide range of fields, and involving both a hardware-level technology and a software-level technology. Basic AI technologies generally include technologies such as a sensor, a dedicated AI chip, cloud computing, distributed storage, a big data processing technology, an operating/interaction system, and electromechanical integration. AI software technologies mainly include fields such as a computer vision technology, a speech processing technology, a natural language processing technology, machine learning/deep learning, automatic driving, and intelligent transportation.

In the field of biotechnologies, by analyzing a binding affinity between a protein and a small molecule, a druggability potential of the small molecule may be analyzed. Therefore, detecting the binding affinity between the protein and the small molecule is a crucial technology.

Generally, a binding affinity detection model may be trained by using structure data of a sample protein and structure data of a sample small molecule, and a binding affinity between a target protein and a target small molecule is detected by using the binding affinity detection model. Since the binding affinity between the protein and the small molecule is closely related to the druggability potential of the small molecule, how to train a binding affinity detection model with higher accuracy becomes an important technology.

An embodiment of the present disclosure provides a method for obtaining a binding affinity detection model. The method may be applied in the foregoing implementation environment and may improve the accuracy of the binding affinity detection model. A flowchart of a method for obtaining a binding affinity detection model according to an embodiment of the present disclosure shown in FIG. 2 is used as an example. For ease of description, the terminal device 101 or the server 102 that performs the method for obtaining a binding affinity detection model in the embodiments of the present disclosure is referred to as an electronic device, and the method may be performed by the electronic device. As shown in FIG. 2, the method includes the following steps:

Step 201: Obtain protein structure data of a sample protein, small molecule structure data of a sample small molecule, and sample labeling data.

The protein is a substance with a certain spatial structure (such as a three-dimensional structure) formed by twisting and folding of a polypeptide chain. The polypeptide chain is a substance formed by amino acids through dehydration and condensation. Since the protein has a certain spatial structure, the protein structure data of the sample protein may be used to represent a spatial structure of the sample protein. A user may input the protein structure data of the sample protein into the electronic device, or another device may send the protein structure data of the sample protein to the electronic device, so that the electronic device obtains the protein structure data of the sample protein.

A sample small molecule is used for manufacturing a drug. Hence, the sample small molecule can be regarded as a sample small drug molecule. A small molecule is a substance whose molecular weight is less than a threshold (for example, the molecular weight is less than 500) and may be configured for making drugs. In all embodiments of the present disclosure, the small molecule may be referred to as a small molecule for short. The small molecule has a certain spatial structure (for example, a three-dimensional structure), and the small molecule structure data of the sample small molecule may be used to represent a spatial structure of the sample small molecule. A user may input the small molecule structure data of the sample small molecule into the electronic device, or another device may send the small molecule structure data of the sample small molecule to the electronic device, so that the electronic device may obtain the small molecule structure data of the sample small molecule.

In all embodiments of the present disclosure, one sample protein and one sample small molecule may be referred to as a sample pair. In the sample pair, a function of the sample protein is affected by interaction between the sample small molecule and the sample protein, and an effect of treating diseases may be achieved through a series of related reactions. The interaction between the sample small molecule and the sample protein may be measured by using a binding affinity (Binding Affinity). For example, the binding affinity is configured for measuring a strength (also referred to as a binding strength) of interaction between a protein and a small molecule. The binding affinity includes data types such as an inhibition constant (referred to as a Ki value for short), a dissociation constant (referred to as a Kd value for short), and a Michaelis constant (referred to as a Km value for short). For example, the Ki value is configured for measuring an action form of the sample small molecule in inhibiting a protein function, and the Kd value is configured for measuring an action form of the sample small molecule in activating a protein function. In general, the sample pair is used as an example, and the binding affinity is configured for measuring the binding strength of the sample protein and the sample small molecule. A greater binding affinity indicates a higher binding strength between the sample protein and the sample small molecule and a greater druggability potential of the small molecule.

In all embodiments of the present disclosure, for a sample pair, a binding affinity between a sample protein and a sample small molecule in the sample pair may be manually labeled to obtain sample labeling data of the sample pair, so that the electronic device obtains the sample labeling data of the sample pair. The sample labeling data is obtained through labeling and is the binding affinity between the sample protein and the sample small molecule. In other words, the sample labeling data is obtained through labeling and is configured for representing the binding affinity between the sample protein and the sample small molecule. A quantity of the sample pairs is at least one, sample proteins and/or sample small molecules in any two sample pairs are different, and the sample proteins and/or sample small molecules refer to: at least one of sample proteins and sample small molecules.

Step 202: determine structure data of a plurality of conformational ensembles by inputting the protein structure data and the small molecule structure data into an initial predictive neural network model.

The protein is used as a receptor, and the small molecule is used as a ligand. The ligand and the receptor form a conformational ensemble through binding, and the conformational ensemble may also be referred to as a binding pose (Binding Pose). A three-dimensional structure of the conformational ensemble is closely related to a three-dimensional structure of the ligand and a three-dimensional structure of the receptor. In all embodiments of the present disclosure, a three-dimensional structure of the sample protein is represented by the protein structure data, and a three-dimensional structure of the sample small molecule is represented by the small molecule structure data. Based on the protein structure data and the small molecule structure data, three-dimensional structures of conformational ensembles formed by binding of the sample protein and the sample small molecule may be obtained through prediction, and the three-dimensional structure of any conformational ensemble is represented by the structure data of the conformational ensemble. A conformational ensemble is a structure obtained through prediction and formed by the binding of the sample protein and the sample small molecule. For example, the conformational ensemble may be determined by the neural network model.

The neural network model in all embodiments of the present disclosure may be an initial network model. The initial network model is an untrained model, that is, a model structure, a size, and the like of the neural network model are the same as a model structure, a size, and the like of the initial network model. The model structure and the size of the initial network model are not limited in the embodiments of the present disclosure. In all embodiments of the present disclosure, the initial predictive neural network model may include, but not limited to, a conformational feature extraction part, a feature fusion part, and a prediction part that are sequentially connected in series. The initial predictive neural network model may further include a data enhancement part. For functions of the parts of the initial network model, reference may be made to related descriptions below and details are not described herein again. Optionally, the neural network model may also be a model obtained by training the initial network model at least once according to the manner from step 201 to step 204, or a model obtained by training the initial network model at least once according to another training manner.

In a possible implementation, step 202 includes step 2021 to step 2023.

Step 2021: Generate structure data of a plurality of candidate conformational ensembles based on the protein structure data and the small molecule structure data.

For example, the protein structure data and the small molecule structure data may be inputted into simulation software, the simulation software is called to predict three-dimensional structures of candidate conformational ensembles formed by the binding of the sample protein and the sample small molecule, and the structure data of the candidate conformational ensembles are outputted, so that the three-dimensional structures of the candidate conformational ensembles are represented by using the structure data of the candidate conformational ensembles. Optionally, the simulation software is docking software. The docking software may simulate and predict a position and an orientation of a small molecule in a protein. For a small molecule and a protein, since there may be a plurality of positions and orientations of the small molecule in the protein, one position and one orientation of the small molecule in the protein correspond to one docking manner of the small molecule and the protein. The docking manner forms a three-dimensional structure of one candidate conformational ensemble. Therefore, the docking software may obtain the three-dimensional structures of the plurality of candidate conformational ensembles by predicting various docking manners of the small molecule and the protein, and only a few of these candidate conformational ensembles may be close to a real binding situation of the small molecule and the protein.

Optionally, the protein structure data includes at least one type; and step 2021 includes: for any type of protein structure data, generating structure data of a candidate conformational ensemble corresponding to the type of protein structure data based on the type of protein structure data and the small molecule structure data; and determining the structure data of the plurality of candidate conformational ensembles based on structure data of candidate conformational ensembles corresponding to various types of protein structure data.

Generally, a three-dimensional structure of a protein is flexible and changes dynamically in a physiological environment. During binding of the protein and a small molecule, the three-dimensional structure of the protein also changes accordingly based on a three-dimensional structure of the small molecule. Based on the foregoing reason, in all embodiments of the present disclosure, for any sample protein, at least one type of protein structure data of the sample protein may be obtained to express various three-dimensional structures of the sample protein by using the at least one type of protein structure data. Through the various three-dimensional structures of the sample protein, three-dimensional structures of candidate conformational ensembles formed by the binding of the sample protein and the sample small molecule that are obtained through prediction are enriched, so that the candidate conformational ensembles more likely cover the real binding situation of the sample protein and the sample small molecule, thereby improving a possibility of correct docking between the sample protein and the sample small molecule.

For any type of protein structure data, the protein structure data and small molecule structure data may be inputted into the simulation software, and the simulation software is called to predict at least one candidate conformational ensemble formed after the sample protein and the sample small molecule are combined based on a three-dimensional structure of a sample protein corresponding to the protein structure data and a three-dimensional structure of a sample small molecule corresponding to the protein structure data, and structure data of the at least one candidate conformational ensemble corresponding to the protein structure data is outputted.

Since any type of protein structure data corresponds to structure data of at least one candidate conformational ensemble, the sample protein corresponds to at least one type of protein structure data. Therefore, structure data of various candidate conformational ensembles corresponding to various types of protein structure data may be collectively referred to as structure data of various (that is, a plurality of) candidate conformational ensembles corresponding to the sample protein.

Step 2022: Call the neural network model to determine quality indicators of the candidate conformational ensembles based on the structure data of the candidate conformational ensembles, where the quality indicator of each candidate conformational ensemble is configured for indicating quality of the candidate conformational ensemble.

In all embodiments of the present disclosure, the structure data of the candidate conformational ensembles may be inputted into the neural network model. The neural network model is used to score, based on the structure data of any candidate conformational ensemble, the quality of the candidate conformational ensemble, to obtain the quality indicator of the candidate conformational ensemble. Optionally, the quality indicator of the candidate conformational ensemble is data that is greater than or equal to 0 and less than or equal to 1. A larger similarity between a candidate conformational ensemble and the real binding situation of the sample protein and the sample small molecule indicates higher quality of the candidate conformational ensemble and a larger quality indicator of the candidate conformational ensemble.

Optionally, step 2022 includes step A1 to step A3.

Step A1: Determine graph structures of the candidate conformational ensembles based on the structure data of the candidate conformational ensembles, where the graph structure of any candidate conformational ensemble is configured for representing a spatial structure of the candidate conformational ensemble.

In all embodiments of the present disclosure, based on the structure data of any candidate conformational ensemble, the graph structure of the candidate conformational ensemble may be determined, and the three-dimensional structure of the candidate conformational ensemble is expressed concisely and vividly by using the graph structure of the candidate conformational ensemble. The graph structure of the candidate conformational ensemble includes a plurality of nodes and a plurality of edges. There is at least one edge on any node and the node is connected to another node through any edge. There may or may not be an edge between any two nodes.

In a possible implementation, the structure data of any candidate conformational ensemble includes atomic data of a plurality of atoms, and any one of the atoms is an atom in the sample protein or an atom in the sample small molecule; and the graph structure of the candidate conformational ensemble includes a plurality of nodes and a plurality of edges. In this case, step A1 includes the following steps: for the candidate conformational ensemble, determining distance data between every two atoms based on the atomic data of the plurality of atoms included in the candidate conformational ensemble; determining the nodes included in the graph structure of the candidate conformational ensemble based on the atomic data of the atoms included in the candidate conformational ensemble; and for any two nodes included in the graph structure of the candidate conformational ensemble, in a case that the distance data between two atoms corresponding to the two nodes is less than a distance threshold, adding an edge between the two nodes.

In a case that the sample protein includes a plurality of atoms, the protein structure data includes atomic data of the plurality of atoms. In a case that the sample small molecule includes a plurality of atoms, the small molecule structure data includes atomic data of the plurality of atoms. Therefore, when at least one candidate conformational ensemble is obtained through the binding of the sample protein and the sample small molecule, structure data of the candidate conformational ensemble includes the atomic data of the atoms in the sample protein and the atomic data of the atoms in the sample small molecule. The atomic data of any atom includes three-dimensional coordinates of the atom, an atom type, and the like. An atom category (that is, type) represents that the atom belongs to an atom in the sample protein or the atom belongs to an atom in the sample small molecule.

For any two atoms included in any candidate conformational ensemble, distance data between the two atoms may be determined according to a distance formula between two points based on three-dimensional coordinates of the two atoms. Next, the atomic data of the atoms included in the candidate conformational ensemble is used as nodes included in the graph structure of the candidate conformational ensemble. In other words, nodes included in the graph structure of any candidate conformational ensemble are determined based on atomic data of atoms included in the candidate conformational ensemble. The atomic data of each atom corresponds to a node in the graph structure of the candidate conformational ensemble. Based on an atom type included in atomic data of an atom, a representation form of a node corresponding to the atomic data of the atom may be determined. When the atom type represents that the atom belongs to an atom in the sample protein, a corresponding node may be represented in a first form (such as black), and when the atom type represents that an atom belongs to an atom in the sample small molecule, a corresponding node may be represented in a second form (such as white), where the first form is different from the second form.

For any two nodes included in the graph structure of the candidate conformational ensemble, in a case that the distance data between two atoms corresponding to the two nodes is less than a distance threshold, an edge is added between the two nodes. In this way, it may be determined whether there is an edge between every two nodes in the graph structure of the candidate conformational ensemble, so that the graph structure of the candidate conformational ensemble is obtained. The distance threshold is not limited in the embodiments of the present disclosure. For example, the distance threshold is 6 angstroms (a unit is Å). Angstrom is a unit of length measurement, a full name is Angstrom, and 1 angstrom is equal to 0.1 nanometer.

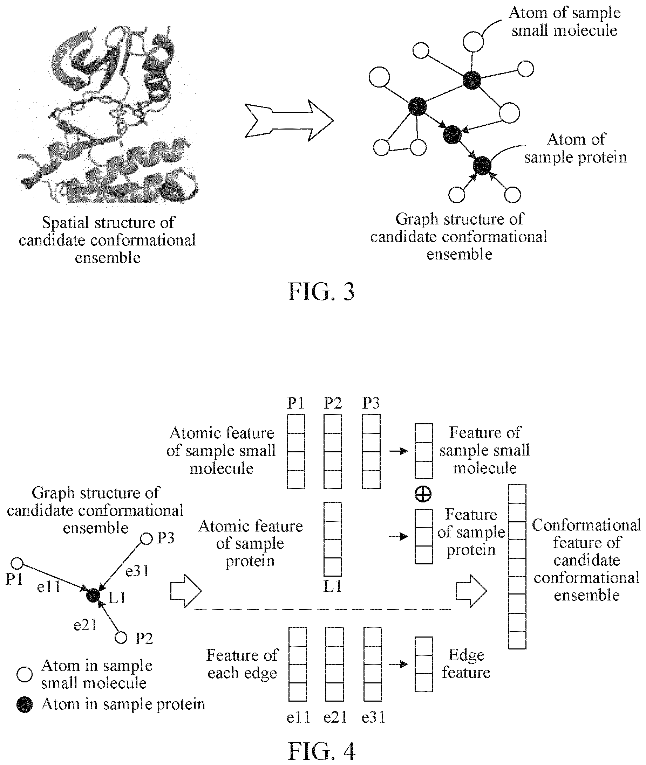

FIG. 3 is a schematic diagram of a spatial structure and a graph structure of a candidate conformational ensemble according to an embodiment of the present disclosure. In all embodiments of the present disclosure, a spatial structure of a candidate conformational ensemble may be converted into a graph structure of the candidate conformational ensemble. It may be seen from the graph structure of the candidate conformational ensemble that the graph structure of the candidate conformational ensemble includes a plurality of nodes and a plurality of edges, and any node is an atom of a sample small molecule or an atom of a sample protein. There is at least one edge on any node and the node is connected to another node through any edge. There may or may not be an edge between any two nodes.

Step A2: Call the neural network model to determine conformational features of the candidate conformational ensembles based on the graph structures of the candidate conformational ensembles.

A graph structure of any candidate conformational ensemble is inputted into the neural network model, and through a conformational feature extraction part of the neural network model, a conformational feature of the candidate conformational ensemble is determined based on the graph structure of the candidate conformational ensemble.

Optionally, the graph structure of the candidate conformational ensemble includes the following information, where N represents a quantity of nodes, D represents content of a node, and L represents a quantity of edges.

GraphData (

x: content of each node (N*D)

edge_index: a list of edges on each node (L*2)

dist: a length of each edge (L*1)

coords: three-dimensional coordinates of each node (N*3)

node_type: a type of each node, where 0 represents that the node belongs to an atom in the sample small molecule, and 1 represents that the node belongs to an atom in the sample protein (N* 1)

tar_lig_id: an identity document (ID) of a sample pair including the sample small molecule and the sample protein

pose_id: an identity document of the candidate conformational ensemble

Rank Index: an identity document of a candidate conformational ensemble set corresponding to a sample pair generated by simulation software, where the candidate conformational ensemble set includes candidate conformational ensembles corresponding to the sample pair

y: sample labeling data

label: a labeled category of the candidate conformational ensemble).

For an edge between a node A and a node B, the edge belongs to both an edge on the node A and an edge on the node B. Therefore, a quantity of edges in the list of edges (that is, a quantity of edges on each node) is two times of the quantity of edges.

For any node, the conformational feature extraction part in the neural network model may perform a pooling operation on at least one of the content of the node, the three-dimensional coordinates of the node, the type of the node, and the like, to extract a feature of the node. In a possible implementation, features of the nodes are used as the conformational feature of the candidate conformational ensemble.

Alternatively, for any edge, the conformational feature extraction part may perform a pooling operation on at least one of respective content, three-dimensional coordinates, types, and the like of two nodes at both ends of the edge, and/or a length of the edge, to extract a feature of the edge. In other words, for any edge, the conformational feature extraction part may perform a pooling operation on at least one of first reference information and a length of the edge. The first reference information includes at least one of respective content, three-dimensional coordinates, types, and the like of two nodes at both ends of the edge. In another possible implementation, features of edges are used as the conformational feature of the candidate conformational ensemble. In still another possible implementation, features of nodes and features of edges are used as the conformational feature of the candidate conformational ensemble.

Optionally, the conformational feature extraction part may perform feature extraction by using a multi-category pooling operation, to obtain the conformational feature of the candidate conformational ensemble. For example, when a type of a node represents that the node belongs to an atom in the sample small molecule or the node belongs to an atom in the sample protein, and feature extraction is performed by using a multi-category pooling operation, for any atom in the sample small molecule or any atom in the sample protein, the multi-category pooling operation is performed on content of the atom, three-dimensional coordinates of the atom, and the like, to obtain an atomic feature of the atom, and the atomic feature of the atom corresponds to the feature of the node. Afterwards, the conformational feature of the candidate conformational ensemble is obtained based on the features of the nodes.

For another example, the graph structure of the candidate conformational ensemble includes a type of an edge. The type of the edge may represent three types of edges, which are respectively an edge between two atoms in the sample small molecule, an edge between two atoms in the sample protein, and an edge between an atom in the sample small molecule and an atom in the sample protein. For any of the foregoing three types of edges, a pooling operation may be performed on at least one of respective content, three-dimensional coordinates, types, and the like of two nodes at both ends of the edge, and/or a length of the edge, to obtain the feature of the edge. In other words, a pooling operation is performed on at least one of second reference information and a length of the edge, to obtain the feature of the edge. The second reference information includes at least one of respective content, three-dimensional coordinates, types, and the like of two nodes at both ends of the edge. Afterwards, the conformational feature of the candidate conformational ensemble is obtained based on the features of the edges.

FIG. 4 is a schematic diagram of extracting a conformational feature of a candidate conformational ensemble according to an embodiment of the present disclosure. The graph structure of the candidate conformational ensemble includes atoms P1 to P3 in the sample small molecule, an atom L1 in the sample protein, an edge e 11 between P1 and L1, an edge e21 between P2 and L1, and an edge e31 between P3 and L1. The conformational feature extraction part may extract an atomic feature of the sample small molecule (that is, atomic features of P1 to P3), an atomic feature of the sample protein (that is, an atomic feature of L1), and features of the edges (that is, features of e11, e21 and e31). The atomic features of P1 to P3 are used as a feature of the sample small molecule, the atomic feature of L1 is used as a feature of the sample protein, and the features of e 11, e21 and e31 are used as edge features. The feature of the sample small molecule, the feature of the sample protein, and the edge features are used as the conformational feature of the candidate conformational ensemble.

An algorithm used by the conformational feature extraction part to extract the conformational feature of the candidate conformational ensemble is not limited in the embodiments of the present disclosure. For example, the algorithm may be a PotentialNet algorithm, a SchNet algorithm, and the like. After the conformational feature extraction part extracts at least one of the features of the nodes and the features of the edges, statistics collection may be further performed on the graph structure of the candidate conformational ensemble to obtain a statistical feature. The statistical feature is configured for representing a quantity of nodes, a quantity of nodes corresponding to each node type, a quantity of edges corresponding to each edge type, and the like in the graph structure of the candidate conformational ensemble. Afterwards, the conformational feature of the candidate conformational ensemble is determined based on at least one of the statistical feature, the features of the nodes, and the features of the edges.

It may be understood that, the foregoing step A1 and step A2 are to first determine the graph structures of the candidate conformational ensembles based on the structure data of the candidate conformational ensembles, and then determine the conformational features of the candidate conformational ensembles based on the graph structures of the candidate conformational ensembles. During application, a three-dimensional convolutional neural network (3D CNN) may be used to directly perform feature extraction on the structure data of the candidate conformational ensembles, to obtain the conformational features of the candidate conformational ensembles.

Step A3: Call the neural network model to determine the quality indicators of the candidate conformational ensembles based on the conformational features of the candidate conformational ensembles.

After the conformational feature extraction part of the neural network model extracts the conformational feature of the candidate conformational ensemble, the quality indicator of the candidate conformational ensemble may be determined based on the conformational feature of the candidate conformational ensemble through a data enhancement part of the neural network model.

Optionally, the data enhancement part of the neural network model includes a multi-layer perceptron (MLP) and an output layer. The multi-layer perceptron is configured for performing down-sampling processing on the conformational feature of the candidate conformational ensemble at least once to obtain a down-sampling feature, where a purpose of the down-sampling processing is to reduce a dimension of the conformational feature of the candidate conformational ensemble. The output layer is configured for mapping the down-sampling feature into the quality indicator of the candidate conformational ensemble, where the mapping processing may be at least one of linear mapping processing and nonlinear mapping processing.

In all embodiments of the present disclosure, the initial predictive neural network model may also perform feature extraction on the protein structure data to obtain a protein feature, and perform feature extraction on the small molecule structure data to obtain a small molecule feature. The multi-layer perceptron may aggregate at least one of the protein feature and the small molecule feature with the conformational feature of the candidate conformational ensemble to obtain an aggregated feature, and perform down-sampling processing on the aggregated feature at least once to obtain a down-sampling feature. Afterwards, the down-sampling feature is mapped into the quality indicator of the candidate conformational ensemble through the output layer.

Step 2023: Call the neural network model to select a plurality of conformational ensembles from the plurality of candidate conformational ensembles based on the quality indicators of the candidate conformational ensembles, to obtain the structure data of the plurality of conformational ensembles.

In step 2022, structure data of a plurality of candidate conformational ensembles may be generated based on the protein structure data and the small molecule structure data. Most conformational ensembles in the plurality of candidate conformational ensembles are different from the real structure formed by the binding of the sample protein and the sample small molecule, and only a few conformational ensembles are relatively consistent with the real structure. In a case that no selection is performed on the plurality of candidate conformational ensembles and the plurality of candidate conformational ensembles are directly used to train the binding affinity detection model, the model has an excessively large calculation amount and poor accuracy. If only one conformational ensemble is selected from the plurality of candidate conformational ensembles, the target conformational ensemble may cover the real structure formed by the binding of the sample protein and the sample small molecule, or may not cover the real structure. As a result, the problem of poor accuracy may also exist.

Therefore, in all embodiments of the present disclosure, some candidate conformational ensembles may be selected from the plurality of candidate conformational ensembles as the plurality of conformational ensembles. Since the candidate conformational ensemble have structure data, and the plurality of conformational ensembles are selected from the candidate conformational ensembles, the plurality of conformational ensembles also have structure data, so that the structure data of the plurality of conformational ensembles can be obtained. For example, a probability that one conformational ensemble covers the real structure is 50%. In a case that three conformational ensembles are selected from the plurality of candidate conformational ensembles, a probability that the three conformational ensembles cover the real structure is 75%. Therefore, a probability of covering the real structure may be increased through the plurality of conformational ensembles, so that the plurality of conformational ensembles can express the real structure more accurately, thereby improving the accuracy of the binding affinity detection model.

After the quality indicators of the candidate conformational ensembles are obtained, a plurality of conformational ensembles whose quality indicator is greater than a reference indicator may be selected from the plurality of candidate conformational ensembles to avoid manual selection of the conformational ensembles. The reference indicator may be set data, for example, the reference indicator is 0.7. Alternatively, the reference indicator may be a set quantity[th] quality indicator after the quality indicators of the candidate conformational ensembles are sorted. For example, the quality indicators of the candidate conformational ensembles are sorted in descending order, and a fourth quality indicator is used as the reference indicator. In this case, candidate conformational ensembles corresponding to the first three quality indicators are selected from the plurality of candidate conformational ensembles as the plurality of conformational ensembles.

Optionally, labeled categories of the candidate conformational ensembles may be determined, where the labeled category of any candidate conformational ensemble is configured for representing whether the candidate conformational ensemble is similar to a benchmark conformational ensemble, and the benchmark conformational ensemble is a real structure formed by the binding of the sample protein and the sample small molecule. After the quality indicators of the candidate conformational ensembles are obtained, a plurality of candidate conformational ensembles that are similar to the benchmark conformational ensemble may be first selected from the plurality of candidate conformational ensembles, and a plurality of conformational ensembles whose quality indicator is greater than the reference indicator are then selected from the plurality of candidate conformational ensembles that are similar to the benchmark conformational ensemble. A manner of determining the labeled categories of the candidate conformational ensembles is similar to a manner of determining labeled categories of the conformational ensembles mentioned below, and details are not described herein.

Step 203: determine a predictive binding affinity between the sample protein and the sample small molecule by inputting the structure data of the conformational ensembles into the initial predictive neural network model.

The sample prediction result is obtained through prediction and is the binding affinity between the sample protein and the sample small molecule. In other words, the sample prediction result is obtained through prediction and is configured for representing the binding affinity between the sample protein and the sample small molecule. The binding affinity between the sample protein and the sample small molecule may be predicted through the neural network model based on the structure data of the conformational ensembles, to obtain the sample prediction result. A larger sample prediction result represents a stronger predicted binding affinity between the sample protein and the sample small molecule and a greater druggability potential of the sample small molecule.

In a possible implementation, step 203 includes step 2031 to step 2032.

Step 2031: Call the neural network model to determine weights of the conformational ensembles based on the conformational features of the conformational ensembles, where the conformational feature of any conformational ensemble is determined based on the structure data of the conformational ensemble.

In all embodiments of the present disclosure, the neural network model may include a feature fusion part. The feature fusion part may determine, based on the conformational feature of any conformational ensemble, a weight of the conformational ensemble. Therefore, the weight of the conformational ensemble is related to the conformational feature of the conformational ensemble. Step A1 and step A2 have mentioned the manner of determining the conformational feature of the candidate conformational ensemble based on the structure data of the candidate conformational ensemble. Since the conformational ensemble is selected from the plurality of candidate conformational ensembles, a manner of determining the conformational feature of the conformational ensemble is similar to the manner of determining the conformational feature of the candidate conformational ensemble, and details are not described herein again.

Optionally, step 2031 includes: calling the neural network model to determine weighted conformational features of the conformational ensembles based on a reference weight and the conformational features of the conformational ensembles; and calling the neural network model to perform normalization processing on the weighted conformational features of the conformational ensembles to obtain the weights of the conformational ensembles.

The feature fusion part in the neural network model includes the reference weight, and the reference weight is a set vector or is obtained by continuously adjusting the set vector. That is, the reference weight is a learnable vector. The feature fusion part may perform weighted fusion on the reference weight and the conformational feature of any conformational ensemble to obtain a weighted fusion result of the conformational ensemble, and obtain a weighted conformational feature of the conformational ensemble based on the weighted fusion result of the target conformational ensemble.

Optionally, the weighted conformational feature of the conformational ensemble is determined according to Formula (1) shown below.

$$s_i = f(qx_i) \qquad \text{Formula (1)}$$

$s_i$ represents a weighted conformational feature of an $i^{th}$ conformational ensemble. $q$ represents the reference weight, $x_i$ represents a conformational feature of the $i^{th}$ conformational ensemble, $qx_i$ represents a weighted fusion result of the $i^{th}$ conformational ensemble, and $f()$ represents a conversion function in a case that the weighted fusion result is converted into the weighted conformational feature of the $i^{th}$ conformational ensemble.

Next, the feature fusion part may perform normalization processing on the weighted conformational features of the conformational ensembles according to a normalization function, to obtain the weights of the conformational ensembles. The normalization function is not limited in the embodiments of the present disclosure. For example, the normalization function is a Softmax function. The Softmax function may compress the weighted conformational feature of the conformational ensemble to a range from 0 to 1, to obtain the weight of the conformational ensemble. Optionally, the weight of the conformational ensemble is determined according to Formula (2) shown below.

$$\alpha_i = softmax(s_i) = \frac{\exp(s_i)}{\sum \exp(s_i)} \qquad \text{Formula (2)}$$

$\alpha_i$ represents a weight of the $i^{th}$ conformational ensemble, *softmax* represents a function symbol of the normalization function, $s_i$ represents the weighted conformational feature of the $i^{th}$ conformational ensemble, exp represents an exponential function using a natural constant e as the base, and $\Sigma$ represents a function symbol of a summation function.

The foregoing Formula (1) and Formula (2) determine a weight of a single conformational ensemble to all conformational ensembles through an attention mechanism. Therefore, the feature fusion part may include an attention network, and the attention network may be a self-attention network or a multi-head attention (Multi-Head Attention) network. In all embodiments of the present disclosure, the reference weight may be obtained through learning based on the attention mechanism.

Step 2032: Call the neural network model to determine the sample prediction result based on the conformational features and the weights of the conformational ensembles.

The feature fusion part in the neural network model may determine the sample prediction result based on the conformational features of the conformational ensembles and the weights of the conformational ensembles.

Optionally, step 2032 includes: calling the neural network model to perform weighted calculation based on the conformational features and the weights of the conformational ensembles to obtain a weighted fusion feature; and calling the neural network model to determine the sample prediction result based on the weighted fusion feature.

The feature fusion part in the neural network model may perform weighted summation on the conformational features and the weights of the conformational ensembles, to obtain the weighted fusion feature. Optionally, the weighted fusion feature is determined according to Formula (3) shown below.

$$a = \sum_{i}^{N} \alpha_i x_i \qquad \text{Formula (3)}$$

$a$ represents a weighted fusion feature, $N$ represents a quantity of the conformational ensembles, $\alpha_i$ represents the weight of the $i^{th}$ conformational ensemble, and $x_i$ represents the conformational feature of the ith conformational ensemble.

Next, the sample prediction result is determined based on the weighted fusion feature through a prediction part of the neural network model. Optionally, the prediction part of the neural network model includes a multi-layer perceptron and an output layer. The multi-layer perceptron is configured for performing down-sampling processing on the weighted fusion feature at least once to obtain a down-sampling feature, where a purpose of the down-sampling processing is to reduce a dimension of the weighted fusion feature. The output layer is configured for mapping the down-sampling feature into the sample prediction result, where the mapping processing may be at least one of linear mapping processing and nonlinear mapping processing.

By determining the weights of the conformational ensembles and performing weighted fusion on the conformational features of the conformational ensembles by using the weights of the conformational ensembles, the accuracy of the weighted fusion feature may be improved, thereby improving the accuracy of the sample prediction result.

In a possible implementation, the neural network model may also perform feature extraction on the protein structure data to obtain a protein feature, and perform feature extraction on the small molecule structure data to obtain a small molecule feature. The multi-layer perceptron may aggregate at least one of the protein feature and the small molecule feature with the weighted fusion feature to obtain an aggregated feature, and perform down-sampling processing on the aggregated feature at least once to obtain a down-sampling feature. Afterwards, the down-sampling feature is mapped into the sample prediction result through the output layer.

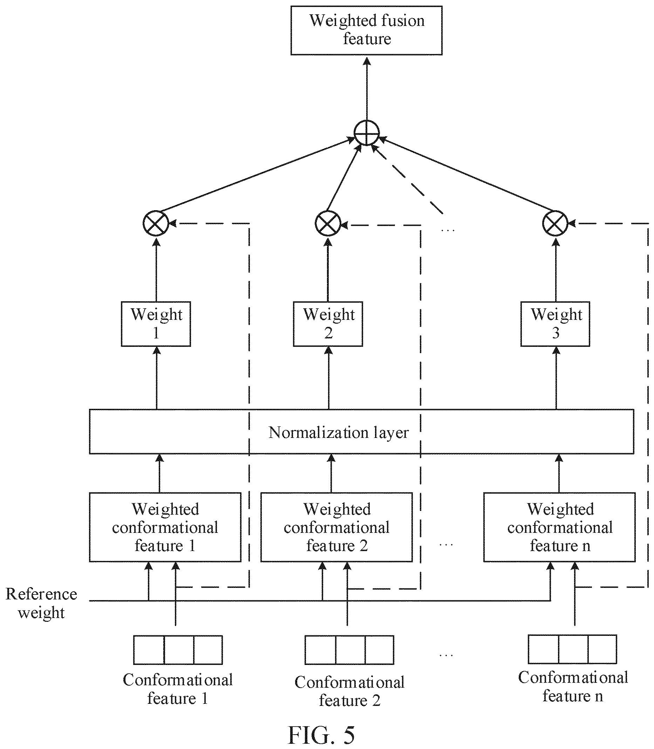

FIG. 5 is a schematic diagram of determining a weighted fusion feature according to an embodiment of the present disclosure. In the embodiments of the present disclosure, there are conformational features of n (n is a positive integer) conformational ensembles, which are respectively denoted as a conformational feature 1 to a conformational feature n. Based on the reference weight and the conformational feature 1 to the conformational feature n, a weighted conformational feature 1 to a weighted conformational feature n may be obtained. Normalization processing is performed on the weighted conformational feature 1 to the weighted conformational feature n by using a normalization layer, to obtain a weight 1 to a weight n. By multiplying the weight 1 and the conformational feature 1, multiplying a weight 2 and a conformational feature 2, and so on, n multiplication results are obtained, and a weighted fusion feature is obtained by adding the n multiplication results.

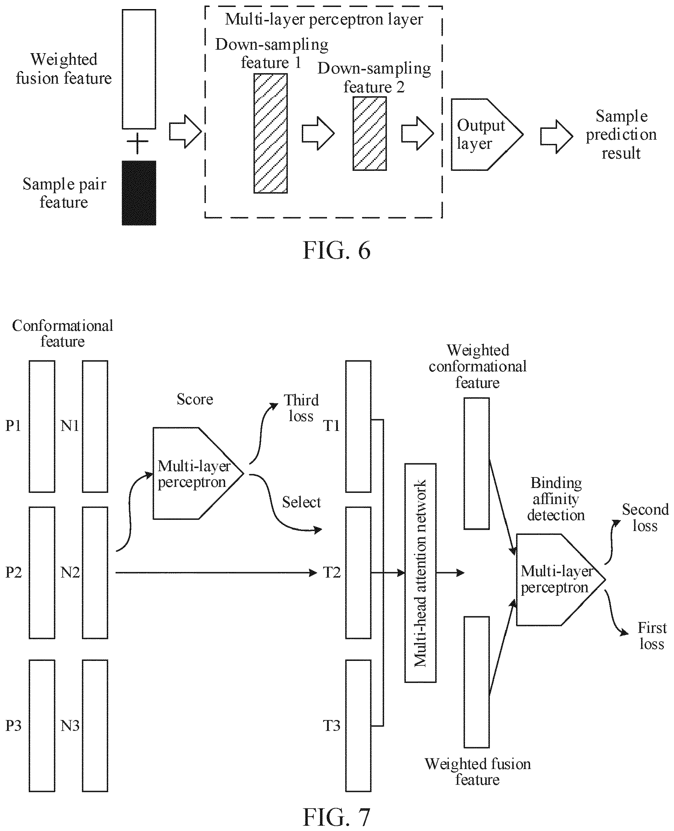

Next, FIG. 6 is a schematic diagram of determining a sample prediction result according to an embodiment of the present disclosure. In all embodiments of the present disclosure, the weighted fusion feature may be spliced with a sample pair feature and then inputted into the multi-layer perceptron, where the sample pair feature include the protein feature and the small molecule feature. The multi-layer perceptron aggregates the protein feature, the small molecule feature, and the weighted fusion feature to obtain an aggregated feature, perform first down-sampling processing on the aggregated feature to obtain a down-sampling feature 1, and then perform second down-sampling processing on the down-sampling feature 1 to obtain a down-sampling feature 2. Afterwards, the down-sampling feature 2 is mapped into the sample prediction result through the output layer.

Step 204: Train the initial neural network model using the sample prediction result and the sample labeling data to obtain the binding affinity detection model.

The binding affinity detection model is configured for detecting a binding affinity between a target protein and a target small molecule. The binding affinity detection model may be applied in a preclinical research and development scenario of drug design, for example, applied in scenarios such as discovery of hit compounds and optimization of lead compounds. During training of the binding affinity detection model, the model may learn a binding mode between a protein and a small molecule, so that the binding affinity between the target protein and the target small molecule can be detected, further biological experimental verification may be performed on a given small molecule with a higher binding affinity, and a result of the biological experimental verification is used to provide feedback and guide to structural optimization of the given small molecule.

Through step 201 to step 203, the sample prediction result and the sample labeling data may be obtained. A loss of the neural network model may be calculated based on the sample prediction result and the sample labeling data, and the neural network model is trained once based on the loss of the neural network model, to obtain a trained neural network model.

In a case that the trained neural network model satisfies a training ending condition, the trained neural network model is used as the binding affinity detection model. The binding affinity detection model is configured for detecting a binding affinity between a target protein and a target small molecule in the manner of step 801 to step 803 mentioned below.

In a case that the trained neural network model does not satisfy the training ending condition, the trained neural network model is used as a neural network model for next training, and the neural network model is trained at least once again in the manner of step 201 to step 204 until the binding affinity detection model is obtained.

Satisfying the training ending condition is not limited in the embodiments of the present disclosure. For example, the satisfying the training ending condition refers to that a quantity of training times of the neural network model reaches a set quantity of times (for example, 500 times). Alternatively, the satisfying the training ending condition refers to that a difference between a loss of a neural network model obtained in last training and a loss of a neural network model obtained in next training is less than a threshold. In other words, in a case that a difference between a loss of a neural network model obtained in previous training of this training and a loss of a neural network model obtained in this training is less than the threshold, it may be considered that the training ending condition is satisfied.

In a possible implementation, step 204 includes step 2041 to step 2044.

Step 2041: Determine a first loss based on the sample prediction result and the sample labeling data.

In a possible implementation, the sample prediction result and the sample labeling data may be subtracted to obtain a difference between the sample prediction result and the sample labeling data, and the first loss is determined based on the difference. For example, at least one of square root operation, exponentiation operation, logarithm operation, and the like is performed on the difference to obtain the first loss.

Step 2042: Determine prediction results of the conformational ensembles based on the structure data of the conformational ensembles, where the prediction result of each conformational ensemble is obtained through prediction and is a binding affinity of the conformational ensemble. In other words, the prediction result of the conformational ensemble is obtained through prediction and is configured for representing the binding affinity of the conformational ensemble. A greater prediction result of the conformational ensemble represents a stronger binding affinity of the conformational ensemble.

The prediction part of the neural network model may determine a prediction result of any conformational ensemble based on the structure data of the conformational ensemble. Optionally, the conformational feature of the conformational ensemble may be first determined based on the structure data of the conformational ensemble, and then, in a manner similar to "determining the sample prediction result based on the weighted fusion feature", the prediction result of the target conformational ensemble is determined based on the conformational feature of the target conformational ensemble.

Optionally, the weights of the conformational ensembles may be further determined based on the conformational features of the conformational ensembles. The weighted conformational feature of the conformational ensemble is determined based on the conformational feature and the weight of the conformational ensemble, and then, in a manner similar to "determining the sample prediction result based on the weighted fusion feature", the prediction result of the conformational ensemble is determined based on the weighted conformational feature of the conformational ensemble.

Step 2043: For any conformational ensemble, determine a second loss corresponding to the conformational ensemble based on the sample labeling data and the prediction result of the conformational ensemble.

In all embodiments of the present disclosure, whether any conformational ensemble is a positive sample or a negative sample may be distinguished based on a comparative learning strategy. In a case that the conformational ensemble is a positive sample, a positive sample formula is used (refer to a formula for determining "the second loss corresponding to the conformational ensemble" in case C1 below), and the second loss corresponding to the conformational ensemble is determined based on the sample labeling data and the prediction result of the conformational ensemble. In a case that the conformational ensemble is a negative sample, a negative sample formula is used (refer to a formula for determining "the second loss corresponding to the conformational ensemble" in case C2 below), and the second loss corresponding to the conformational ensemble is determined based on the sample labeling data and the prediction result of the conformational ensemble.

Optionally, step 2043 includes step B1 to step B3.

Step B 1: Obtain structure data of a benchmark conformational ensemble.

The benchmark conformational ensemble is a real structure formed by the binding of the sample protein and the sample small molecule, and has a certain three-dimensional structure. In all embodiments of the present disclosure, the structure data of the benchmark conformational ensemble may be obtained by analyzing the three-dimensional structure of the benchmark conformational ensemble, and the three-dimensional structure of the benchmark conformational ensemble is represented by using the structure data of the benchmark conformational ensemble.

Step B2: Determine a labeled category of the conformational ensemble based on the structure data of the benchmark conformational ensemble and the structure data of the conformational ensemble, where the labeled category of the conformational ensemble is configured for representing whether the conformational ensemble is similar to the benchmark conformational ensemble.

In a possible implementation, feature extraction may be performed on the structure data of the benchmark conformational ensemble to obtain a conformational feature of the benchmark conformational ensemble. Similarly, feature extraction may be performed on the structure data of any conformational ensemble to obtain the conformational feature of the conformational ensemble. For a manner of determining the conformational feature of the benchmark conformational ensemble and the conformational feature of the conformational ensemble, reference may be made to the above-mentioned manner of determining the conformational feature of the candidate conformational ensemble. Implementation principles of the two manners are similar and details are not described herein again.

Next, based on the conformational feature of the benchmark conformational ensemble and the conformational feature of the conformational ensemble, a similarity between the benchmark conformational ensemble and the conformational ensemble may be determined according to a similarity formula. In a case that the similarity between the benchmark conformational ensemble and the conformational ensemble is greater than a similarity threshold, it is determined that the labeled category of the conformational ensemble is that the conformational ensemble is similar to the benchmark conformational ensemble. In a case that the similarity between the benchmark conformational ensemble and the conformational ensemble is not greater than the similarity threshold, it is determined that the labeled category of the conformational ensemble is that the conformational ensemble is not similar to the benchmark conformational ensemble. The similarity formula and the similarity threshold are not limited in the embodiments of the present disclosure. For example, the similarity formula may be a distance formula, a cosine formula, a Jaccard formula, and the like. The similarity threshold is data greater than 0 and less than 1, and for example, the similarity threshold is 0.6.

In another possible implementation, the structure data of the benchmark conformational ensemble includes atomic data of a plurality of atoms. Similarly, the structure data of any conformational ensemble includes atomic data of a plurality of atoms. Since both the benchmark conformational ensemble and the conformational ensemble are structures formed by the binding of the sample protein and the sample small molecule, the binding may change three-dimensional coordinates of an atom, but may not change a type of the atom, and a quantity of atoms. Therefore, atoms in the benchmark conformational ensemble are in a one-to-one correspondence with atoms in the conformational ensemble.

Based on three-dimensional coordinates of any atom in the benchmark conformational ensemble and three-dimensional coordinates of a corresponding atom in the conformational ensemble, a distance between the atom in the benchmark conformational ensemble and the corresponding atom in the conformational ensemble may be calculated. Distances between the atoms in the benchmark conformational ensemble and the corresponding atoms in the conformational ensemble are integrated to obtain a distance between the benchmark conformational ensemble and the conformational ensemble. Alternatively, the distance between the benchmark conformational ensemble and the conformational ensemble may be obtained by performing root mean square deviation (RMSD) calculation based on three-dimensional coordinates of the atoms in the benchmark conformational ensemble and three-dimensional coordinates of the corresponding atoms in the conformational ensemble.

In a case that the distance between the benchmark conformational ensemble and the conformational ensemble is greater than a benchmark distance, it is determined that the labeled category of the conformational ensemble is that the conformational ensemble is not similar to the benchmark conformational ensemble. In a case that the distance between the benchmark conformational ensemble and the conformational ensemble is not greater than the benchmark distance, it is determined that the labeled category of the conformational ensemble is that the conformational ensemble is similar to the benchmark conformational ensemble. The benchmark distance is not limited in the embodiments of the present disclosure, and may be set based on application scenarios or experience. For example, the benchmark distance is 2Å.

Step B3: Determine the second loss corresponding to the conformational ensemble based on the sample labeling data, and the prediction result and the labeled category of the conformational ensemble.

In a case that the labeled category of the conformational ensemble is that the conformational ensemble is similar to the benchmark conformational ensemble, the conformational ensemble is a positive sample, a positive sample formula may be used (refer to a formula for determining "the second loss corresponding to the conformational ensemble" in case C1 below), and the second loss corresponding to the conformational ensemble is determined based on the sample labeling data and the prediction result of the conformational ensemble. In a case that the labeled category of the conformational ensemble is that the conformational ensemble is not similar to the benchmark conformational ensemble, the conformational ensemble is a negative sample, a negative sample formula may be used (refer to a formula for determining "the second loss corresponding to the conformational ensemble" in case C2 below), and the second loss corresponding to the conformational ensemble is determined based on the sample labeling data and the prediction result of the target conformational ensemble.

Optionally, step B3 includes two cases, respectively denoted as case C1 and case C2.

Case C1: In a case that the labeled category of the conformational ensemble is configured for representing that the conformational ensemble is similar to the benchmark conformational ensemble, determine the second loss corresponding to the conformational ensemble based on a target difference between the prediction result of the conformational ensemble and the sample labeling data.

When the conformational ensemble is a positive sample, the target difference is determined according to Formula (4) shown below.

$$diff = pred - true \text{ , positive sample} \qquad \text{Formula (4)}$$

*diff* represents the target difference between the prediction result of the conformational ensemble and the sample labeling data, *pred* represents the prediction result of the conformational ensemble, and *true* represents the sample labeling data.

Next, at least one of square root operation, exponentiation operation, logarithm operation, and the like is performed on the target difference to obtain the second loss corresponding to the conformational ensemble.

The second loss of the positive sample is determined in the manner of case C1, which makes a prediction result of the positive sample continuously approach the sample labeling data, and improves the accuracy of the prediction result of the positive sample, thereby improving the accuracy of the binding affinity detection model.

Case C2: In a case that the labeled category of the conformational ensemble is configured for representing that the conformational ensemble is not similar to the benchmark conformational ensemble, determine the second loss corresponding to the conformational ensemble based on a maximum value of the target difference and a set value.

A magnitude of the set value is not limited in the embodiments of the present disclosure. An example in which the set value is 0 is used. When the conformational ensemble is a negative sample, the maximum value between the target difference and the set value may be determined according to Formula (5) shown below.

$$diff = \max(pred - true, 0), \text{ negative sample} \qquad \text{Formula (5)}$$

*diff* represents the maximum value of the target difference and the set value, *pred-true* represents the target difference between the prediction result of the conformational ensemble and the sample labeling data, *pred* represents the prediction result of the conformational ensemble, and *true* represents the sample labeling data. max represents a function symbol of a maximum function.

At least one of square root operation, exponentiation operation, logarithm operation, and the like is performed on the maximum value of the target difference and the set value to obtain the second loss corresponding to the conformational ensemble.

The second loss of a negative sample is determined in the manner of case C2, and when the prediction result of the negative sample is greater than the sample labeling data, a penalty is imposed on the negative sample, so that the prediction result of the negative sample is less than or equal to the sample labeling data. When the neural network model is subsequently trained based on the second loss corresponding to the negative sample, the trained neural network model may output a smaller prediction result for a negative sample, which improves a perception capability of the model for negative samples, and facilitates the model to distinguish positive samples and negative samples. Therefore, when the second loss of the negative sample is determined in the manner of case C2 and the binding affinity detection model is obtained by training based on the second loss corresponding to the negative sample, the binding affinity detection model has relatively high sensitivity to the conformational ensemble and can more accurately distinguish positive samples and negative samples, so that the binding affinity detection model has relatively high accuracy.

Step 2044: Train the neural network model to obtain the binding affinity detection model based on the first loss and second losses corresponding to the conformational ensembles.

For example, the second losses corresponding to the conformational ensembles may be added to obtain a total second loss. Weighted summation operation is performed on the first loss and the total second loss to obtain a loss of the neural network model, and the neural network model is trained based on the loss of the neural network model to obtain the binding affinity detection model.

In another possible implementation, step 204 includes step 2045 to step 2048.

Step 2045: Determine a first loss based on the sample prediction result and the sample labeling data. For an implementation of step 2045, reference may be made to the description of step 2041 above, and details are not described herein again.

Step 2046: Obtain labeled categories of the candidate conformational ensembles, where the labeled category of each candidate conformational ensemble is configured for representing whether the candidate conformational ensemble is similar to the benchmark conformational ensemble.

The labeled category of any candidate conformational ensemble may be determined based on the structure data of the benchmark conformational ensemble and the structure data of the candidate conformational ensemble. A manner of determining the labeled categories of the candidate conformational ensembles is similar to the manner of determining the labeled categories of the conformational ensembles. Reference may be made to the description of step B2 above, and details are not described herein again.

Step 2047: For any candidate conformational ensemble, determine a third loss corresponding to the candidate conformational ensemble based on the labeled category and the quality indicator of the candidate conformational ensemble.

In the embodiments of the present disclosure, in a case that the labeled category of any candidate conformational ensemble represents that the candidate conformational ensemble is similar to the benchmark conformational ensemble, it is determined that a labeled indicator of the candidate conformational ensemble is first data (such as 1), and based on the first data and the quality indicator of the candidate conformational ensemble, the third loss corresponding to the candidate conformational ensemble is calculated according to a cross-entropy loss function.

In a case that the labeled category of any candidate conformational ensemble represents that the candidate conformational ensemble is not similar to the benchmark conformational ensemble, it is determined that a labeled indicator of the candidate conformational ensemble is second data (such as 0), and based on the second data and the quality indicator of the candidate conformational ensemble, the third loss corresponding to the candidate conformational ensemble is calculated according to a cross-entropy loss function.

Step 2048: Train the neural network model to obtain the binding affinity detection model based on the first loss and third losses corresponding to the candidate conformational ensembles.

For example, the third losses corresponding to the candidate conformational ensembles may be added to obtain a total third loss. Weighted summation operation is performed on the first loss and the total third loss to obtain a loss of the neural network model, and the neural network model is trained based on the loss of the neural network model to obtain the binding affinity detection model.

In a possible implementation, according to Formula (6) shown below, weighted summation operation is performed on the first loss, the total second loss, and the total third loss to obtain a loss of the neural network model, and the neural network model is trained based on the loss of the neural network model to obtain the binding affinity detection model.

$$\mathcal{L}_{loss} = \mathcal{L}_{MSE}^{(sample)} + \alpha * \mathcal{L}_{MSE}^{(pose)} + \beta * \mathcal{L}_{BCE}^{(pose)} \qquad \text{Formula (6)}$$

$\mathcal{L}_{loss}$ represents the loss of the neural network model, $\mathcal{L}_{MSE}^{(sample)}$ represents the first loss, $\mathcal{L}_{MSE}^{(pose)}$ represents the total second loss, $\mathcal{L}_{BCE}^{(pose)}$ represents the total third loss, $\alpha$ represents a weight of the total second loss, and $\beta$ represents a weight of the total third loss.

FIG. 7 is a schematic diagram of obtaining a binding affinity detection model according to an embodiment of the present disclosure. The binding affinity detection model may be obtained by training the neural network model. The neural network model includes two multi-layer perceptrons and a multi-head attention network.

In the embodiments of the present disclosure, for a plurality of candidate conformational ensembles formed by the binding of the sample protein and the sample small molecule, labeled categories of the candidate conformational ensembles are determined. In a case that the labeled category of a candidate conformational ensemble is that the candidate conformational ensemble is similar to a benchmark conformational ensemble, the candidate conformational ensemble is a positive sample. In a case that the labeled category of a candidate conformational ensemble is that the candidate conformational ensemble is not similar to the benchmark conformational ensemble, the candidate conformational ensemble is a negative sample.

Feature extraction is performed on structure data of positive samples to obtain conformational features of the positive samples, which are respectively denoted as P1 to P3. Similarly, feature extraction is performed on structure data of negative samples to obtain conformational features of the positive samples, which are respectively denoted as N1 to N3. P1 to P3 and N1 to N3 are scored by using the multi-layer perceptron to obtain quality indicators of the candidate conformational ensembles. Based on the quality indicators of the candidate conformational ensembles, a plurality of conformational ensembles are selected from the positive samples or from the positive samples and the negative samples.

Conformational features of the conformational ensembles are respectively denoted as T1 to T3, and T1 to T3 are all inputted to the multi-head attention network. On one hand, the multi-head attention network outputs weighted conformational features of the conformational ensembles. The weighted conformational features of the conformational ensembles may be inputted into the multi-layer perceptron for binding affinity prediction, to obtain prediction results of the conformational ensembles. The prediction result of each conformational ensemble is obtained through prediction and is a binding affinity of the conformational ensemble. On the other hand, the multi-head attention network outputs a weighted fusion feature. The weighted fusion feature may be inputted into the multi-layer perceptron for binding affinity prediction to obtain a sample prediction result. The sample prediction result is obtained through prediction and is a binding affinity between a sample protein and a sample small molecule.

In addition, sample labeling data may also be obtained. The sample labeling data is obtained through labeling and is the binding affinity between the sample protein and the sample small molecule. On one hand, a first loss is determined based on the sample prediction result and the sample labeling data. On another hand, second losses corresponding to the conformational ensembles are determined based on the sample labeling data and the prediction results of the conformational ensembles. On still another hand, third losses corresponding to the candidate conformational ensembles are determined based on the labeled categories and the quality indicators of the candidate conformational ensembles.

Next, a loss of the neural network model is determined based on the first loss, the second losses corresponding to the conformational ensembles, and the third losses corresponding to the candidate conformational ensembles. Based on the loss of the neural network model, the neural network model is trained to obtain the binding affinity detection model.

The information (including, but not limited to, user equipment information, user personal information, and the like), data (including, but not limited to, data for analysis, stored data, displayed data, and the like), and signals involved in the present disclosure all are authorized by the user or fully authorized by each party, and the collection, use, and processing of relevant data need to comply with relevant laws and regulations of relevant countries and regions. For example, the protein structure data of the sample protein and the small molecule structure data of the sample small molecule involved in the present disclosure are obtained with full authorization.

The foregoing method is to call the neural network model to determine the structure data of the plurality of conformational ensembles based on the protein structure data of the sample protein and the small molecule structure data of the sample small molecule. Any conformational ensemble is a structure obtained through prediction and formed by binding of a sample protein and a sample small molecule. Through a plurality of conformational ensembles, a probability of covering a real structure formed by the binding of the sample protein and the sample small molecule may be improved. That is, the plurality of conformational ensembles can more accurately express the real structure formed by the binding of the sample protein and the sample small molecule, so that a sample prediction result is more accurate when a neural network model is called to determine the sample prediction result based on structure data of the conformational ensembles. In this way, when the neural network model is trained by using the sample prediction result to obtain a binding affinity detection model, the accuracy of the binding affinity detection model can be improved, thereby improving the accuracy of a binding affinity detection result.

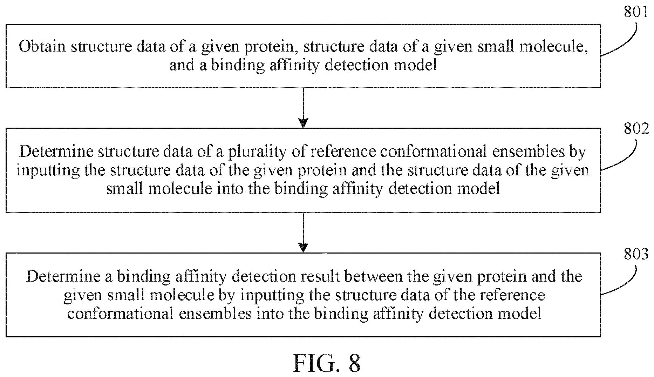

An embodiment of the present disclosure provides a binding affinity detection method, and the method may be applied in the foregoing implementation environment. A flowchart of a binding affinity detection method according to an embodiment of the present disclosure shown in FIG. 8 is used as an example. For ease of description, the terminal device 101 or the server 102 that performs the binding affinity detection method in this embodiment of the present disclosure is referred to as an electronic device, and the method may be performed by the electronic device. As shown in FIG. 8, the method includes the following steps:

Step 801: Obtain protein structure data of a given protein, structure data of a given small molecule, and a binding affinity detection model.

The binding affinity detection model is trained according to the method for obtaining the binding affinity detection model related to FIG. 2. For a manner of obtaining the structure data of the target protein and the structure data of the target small molecule, reference may be made to the description of step 201. Implementation principles of the two manners are similar and details are not described herein again.

Step 802: determine structure data of a plurality of reference conformational ensembles by inputting the structure data of the given protein and the structure data of the given small molecule into the binding affinity detection model.

The reference conformational ensemble is a structure obtained through prediction and formed by binding of the target protein and the target small molecule. For description of step 802, reference may be made to the description of step 202 above. Implementation principles of the two steps are similar, and details are not described herein again.

In a possible implementation, step 802 includes step 8021 to step 8023.

Step 8021: Generate structure data of a plurality of first conformational ensembles based on the structure data of the target protein and the structure data of the target small molecule. For description of step 8021, reference may be made to the description of step 2021 above. Implementation principles of the two steps are similar, and details are not described herein again.

Step 8022: determine quality indicators of first conformational ensembles by inputting the structure data of the first conformational ensembles into the binding affinity detection model, where the quality indicator of each first conformational ensemble is configured for indicating quality of the first conformational ensemble. For description of step 8022, reference may be made to the description of step 2022 above. Implementation principles of the two steps are similar, and details are not described herein again.

Step 8023: select a plurality of reference conformational ensembles from the plurality of first conformational ensembles by inputting the quality indicators of the first conformational ensembles into the binding affinity detection model, to obtain the structure data of the plurality of reference conformational ensembles. For description of step 8023, reference may be made to the description of step 2023 above. Implementation principles of the two steps are similar, and details are not described herein again.

Step 803: determine a binding affinity detection result between the given protein and the given small molecule by inputting the structure data of the reference conformational ensembles into the binding affinity detection model.

The target detection result is a detected binding affinity between the target protein and the target small molecule. In other words, the target detection result is obtained through prediction and is configured for representing the binding affinity between the target protein and the target small molecule. For description of step 803, reference may be made to the description of step 203 above. Implementation principles of the two steps are similar, and details are not described herein again.

In a possible implementation, step 803 includes step 8031 to step 8032.

Step 8031: determine weights of the reference conformational ensembles by inputting conformational features of the reference conformational ensembles into between the given protein and the given small molecule, where the conformational feature of any reference conformational ensemble is determined based on the structure data of the reference conformational ensemble. For description of step 8031, reference may be made to the description of step 2031 above. Implementation principles of the two steps are similar, and details are not described herein again.

Step 8032: determine the binding affinity detection result by inputting the conformational features and the weights of the reference conformational ensembles into between the given protein and the given small molecule. For description of step 8032, reference may be made to the description of step 2032 above. Implementation principles of the two steps are similar, and details are not described herein again.

The information (including, but not limited to, user equipment information, user personal information, and the like), data (including, but not limited to, data for analysis, stored data, displayed data, and the like), and signals involved in the present disclosure all are authorized by the user or fully authorized by each party, and the collection, use, and processing of relevant data need to comply with relevant laws and regulations of relevant countries and regions. For example, the protein structure data of the target protein and the structure data of the target small molecule involved in the present disclosure are obtained with full authorization.

The binding affinity detection model in the foregoing method determines the structure data of the plurality of reference conformational ensembles based on the structure data of the target protein and the structure data of the target small molecule. Any reference conformational ensemble is a structure obtained through prediction and formed by binding of the target protein and the target small molecule. Through a plurality of reference conformational ensembles, a probability of covering a real structure formed by the binding of the target protein and the target small molecule may be improved. That is, the plurality of reference conformational ensembles can more accurately express the real structure formed by the binding of the target protein and the target small molecule, so that a target detection result is more accurate when the binding affinity detection model is called to determine the target detection result based on the structure data of the reference conformational ensembles. That is, the accuracy of a binding affinity detection result is improved.

The foregoing describes the method for obtaining a binding affinity detection model and the binding affinity detection method provided in the embodiments of the present disclosure from a perspective of method steps, and the following systematically describes a binding affinity detection process provided in the embodiments of the present disclosure.

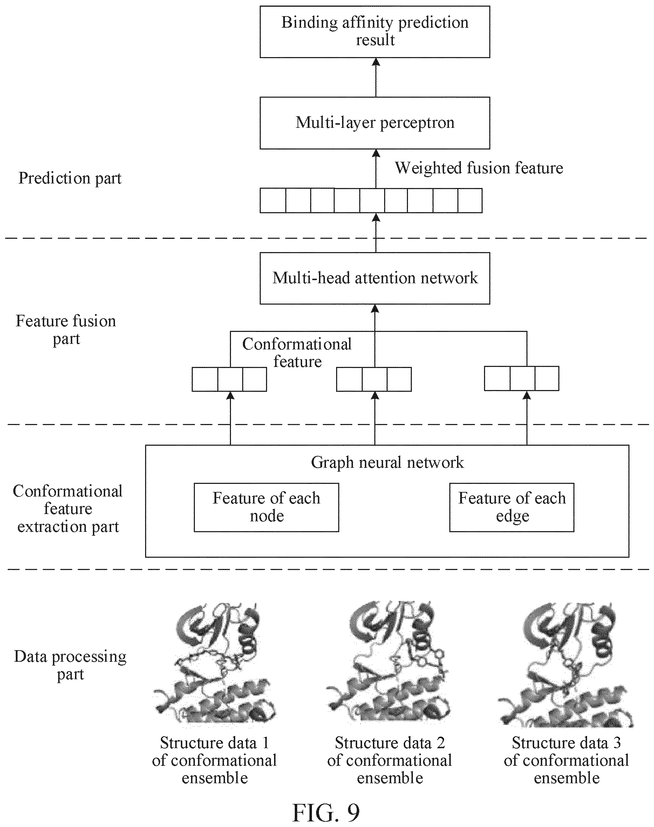

FIG. 9 is a schematic diagram of a binding affinity detection process according to an embodiment of the present disclosure. The binding affinity detection process is divided into four parts, which are respectively a data processing part, a conformational feature extraction part, a feature fusion part, and a prediction part. The following separately describes the four parts:

Since a manner of determining a sample prediction result based on a sample protein and a sample small molecule is similar to a manner of determining a target detection result based on a target protein and a target small molecule, this embodiment of the present disclosure briefly describes that a binding affinity prediction result is determined based on a protein and a small molecule.

The data processing part is configured for determining structure data of a plurality of conformational ensembles based on structure data of the protein and structure data of the small molecule, which are respectively denoted as structure data 1 of a conformational ensemble to structure data 3 of a conformational ensemble. Next, the structure data of the conformational ensembles are converted into graph structures of the conformational ensembles. In addition, the data processing part may also determine labeled categories of the conformational ensembles.

The conformational feature extraction part includes a graph neural network (GNN). The graph neural network may perform feature extraction on the graph structures of the conformational ensembles to obtain conformational features of the conformational ensembles. For any conformational ensemble, features of nodes and features of edges of the conformational ensemble may be extracted from the graph structure of the conformational ensemble. The conformational features of the conformational ensembles are obtained based on the features of the nodes and the features of the edges.

The feature fusion part includes a multi-head attention network, and the multi-head attention network is configured for determining a weighted fusion feature based on the conformational features of the conformational ensembles.

The prediction part includes a multi-layer perceptron, and the multi-layer perceptron is configured for determining the binding affinity prediction result based on the weighted fusion feature.

A public data set may be obtained in the embodiments of the present disclosure. On one hand, an open source binding affinity detection model 1 (that is, a binding affinity detection model in the related art) is obtained. The binding affinity detection model may be a Gnina model. On the other hand, a binding affinity detection model is trained by using the public data set based on the method for obtaining a binding affinity detection model related to FIG. 2, to respectively obtain a binding affinity detection model 2 and a binding affinity detection model 3. The binding affinity detection model 2 uses the first loss as the loss of the neural network model and is obtained through training by using the loss of the neural network model, while the binding affinity detection model 3 performs weighted summation on the first loss, the total second loss, and the total third loss to obtain the loss of the neural network model and is obtained through training by using the loss of the neural network model. Therefore, data enhancement is not performed in the binding affinity detection model 2, and data enhancement is performed in the binding affinity detection model 3.

According to the embodiments of the present disclosure, the public data set is further used to test the detection accuracy of binding affinity detection models 1 to 3, to obtain results shown in Table 1 below.

Table 1

| Model | Pearson | RMSE | AUC |
|---|---|---|---|
| Binding affinity detection model 1 | 0.575 | / | 0.901 |
| Binding affinity detection model 2 | 0.574 | 1.661 | 0.857 |
| Binding affinity detection model 3 | 0.578 | 1.495 | 0.913 |

A pearson correlation coefficient (Pearson for short in Table 1) is a correlation degree between a true value of a binding affinity and a detected value of the binding affinity outputted by the binding affinity detection model. A higher Pearson indicates higher detection accuracy of the binding affinity detection models 1 to 3. A root mean square error (RMSE) is a root mean square error between the true value of the binding affinity and the detected value of the binding affinity outputted by the binding affinity detection model. A lower RMSE indicates higher detection accuracy of the binding affinity detection models 1 to 3. An area under curve (AUC) is an indicator for evaluating a labeled category of a conformational ensemble. A higher AUC indicates a more accurate labeled category of the conformational ensemble. It may be seen from Table 1 that, the detection accuracy of the binding affinity detection model that is trained based on the method for obtaining a binding affinity detection model related to FIG. 2 is higher, and training the binding affinity detection model in a data enhancement manner can further improve the detection accuracy of the model.

A self-built data set may also be obtained in all embodiments of the present disclosure. Since the self-built data set is prone to a data deviation compared with the public data set, an evaluation indicator of the self-built data set is easily excessively high. However, the self-built data set may cover more proteins (including mainstream proteins, new proteins, and the like), and binding of these proteins and small molecules better meets real application scenarios. In all embodiments of the present disclosure, the self-built data set may include three proteins, which are respectively represented by Kinase, DUD-E, and Novel. A binding affinity detection model is trained by using the self-built data set based on the method for obtaining a binding affinity detection model related to FIG. 2, to respectively obtain a binding affinity detection model 4 and a binding affinity detection model 5. Data enhancement is not performed in the binding affinity detection model 4, and data enhancement is performed in the binding affinity detection model 5.

According to the embodiments of the present disclosure, the self-built data set is further used to test the detection accuracy of the binding affinity detection models 1, 4, and 5. For the three proteins Kinase, DUD-E, and Novel, Pearsons of the binding affinity detection models 1, 4, and 5 are respectively calculated to use the Pearson of each protein to locally evaluate the detection accuracy. In addition, for each binding affinity detection model, the Pearsons of the three proteins corresponding to the binding affinity detection model is used to calculate an average value to evaluate the detection accuracy as a whole. Obtained results are shown in Table 2 below.

Table 2

| Model | Kinase | DUD-E | Novel | Average value |
|---|---|---|---|---|
| Binding affinity detection model 1 | 0.230 | 0.249 | 0.212 | 0.231 |
| Binding affinity detection model 4 | 0.273 | 0.259 | 0.187 | 0.223 |
| Binding affinity detection model 5 | 0.286 | 0.288 | 0.212 | 0.249 |

It may be seen from Table 2 that, the detection accuracy of the binding affinity detection model that is trained based on the method for obtaining a binding affinity detection model related to FIG. 2 is higher, and training the binding affinity detection model in a data enhancement manner can further improve the detection accuracy of the model.

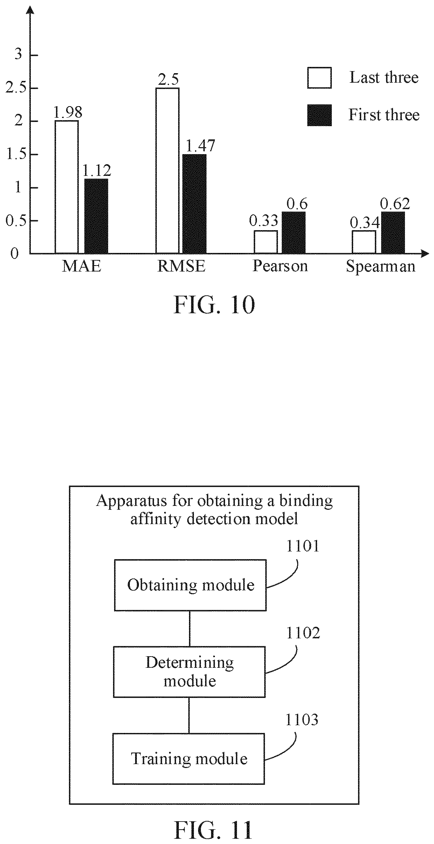

In all embodiments of the present disclosure, the binding affinity detection model 3 may also be used to determine quality indicators of candidate conformational ensembles, and the quality indicators of the candidate conformational ensembles are sorted. On one hand, the first three candidate conformational ensembles after sorting are selected to test values of the binding affinity detection model 3 on four test indicators, namely, a mean absolute error (MAE), the RMSE, the Pearson, and a Spearman correlation coefficient (Spearman). On the other hand, the last three candidate conformational ensembles after sorting are selected to test values of the binding affinity detection model 3 on the four test indicators of the MAE, the RMSE, the Pearson, and the Spearman. Finally, a schematic diagram of comparison of test indicators shown in FIG. 10 is obtained.

It may be seen from FIG. 10 that, for the two test indicators of the MAE and the RMSE, test indicators of the last three candidate conformational ensembles after sorting (referred to as the last three) are both reduced by more than 0.8 (reduced by nearly 40%) compared with test indicators of the first three candidate conformational ensembles before sorting (referred to as the first three). For the two test indicators of the Pearson and the Spearman, compared with test indicators of the first three, test indicators of the last three are increased by about 0.3 (increased by nearly 80%). This indicates that data enhancement may improve the sensitivity of the model for conformational ensembles, so that the detection accuracy of the model is improved.

FIG. 11 is a schematic structural diagram of an apparatus for obtaining a binding affinity detection model according to an embodiment of the present disclosure. As shown in FIG. 11, the apparatus includes:

an obtaining module 1101, configured to obtain protein structure data of a sample protein, small molecule structure data of a sample small molecule, and sample labeling data, where the sample labeling data is obtained through labeling and is a binding affinity between the sample protein and the sample small molecule (in other words, the sample labeling data is obtained through labeling and is configured for representing a binding affinity between the sample protein and the sample small molecule);

a determining module 1102, configured to call a neural network model to determine structure data of a plurality of conformational ensembles based on the protein structure data and the small molecule structure data, where any conformational ensemble is a structure obtained through prediction and formed by binding of the sample protein and the sample small molecule;

the determining module 1102, further configured to call the neural network model to determine a sample prediction result based on the structure data of the conformational ensembles, where the sample prediction result is obtained through prediction and is the binding affinity between the sample protein and the sample small molecule (in other words, the sample prediction result is obtained through prediction and is configured for representing a binding affinity between the sample protein and the sample small molecule); and

a training module 1103, configured to train the neural network model by using the sample prediction result and the sample labeling data to obtain a binding affinity detection model, where the binding affinity detection model is configured for detecting a binding affinity between a target protein and a target small molecule.

In a possible implementation, the determining module 1102 is configured to generate structure data of a plurality of candidate conformational ensembles based on the protein structure data and the small molecule structure data; call the neural network model to determine quality indicators of the candidate conformational ensembles based on the structure data of the candidate conformational ensembles, where the quality indicator of each candidate conformational ensemble is configured for indicating quality of the candidate conformational ensemble; and call the neural network model to select a plurality of conformational ensembles from the plurality of candidate conformational ensembles based on the quality indicators of the candidate conformational ensembles, to obtain the structure data of the plurality of conformational ensembles.

In a possible implementation, the protein structure data includes at least one type; and

the determining module 1102 is configured to, for any type of protein structure data, generate structure data of a candidate conformational ensemble corresponding to the type of protein structure data based on the type of protein structure data and the small molecule structure data; and determine the structure data of the plurality of candidate conformational ensembles based on structure data of candidate conformational ensembles corresponding to various types of protein structure data.

In a possible implementation, the determining module 1102 is configured to determine graph structures of the candidate conformational ensembles based on the structure data of the candidate conformational ensembles, where the graph structure of any candidate conformational ensemble is configured for representing a spatial structure of the candidate conformational ensemble; call the neural network model to determine conformational features of the candidate conformational ensembles based on the graph structures of the candidate conformational ensembles; and call the neural network model to determine the quality indicators of the candidate conformational ensembles based on the conformational features of the candidate conformational ensembles.

In a possible implementation, the structure data of any candidate conformational ensemble includes atomic data of a plurality of atoms, and any one of the atoms is an atom in the sample protein or an atom in the sample small molecule; the graph structure of the candidate conformational ensemble includes a plurality of nodes and a plurality of edges; and

the determining module 1102 is configured to, for the candidate conformational ensemble, determine distance data between every two atoms based on the atomic data of the plurality of atoms included in the candidate conformational ensemble; use the atomic data of atoms included in the candidate conformational ensemble as the nodes included in the graph structure of the candidate conformational ensemble (in other words, determine the nodes included in the graph structure of the candidate conformational ensemble based on the atomic data of the atoms included in the candidate conformational ensemble); and for any two nodes included in the graph structure of the candidate conformational ensemble, in a case that the distance data between two atoms corresponding to the two nodes is less than a distance threshold, add an edge between the two nodes.

In a possible implementation, the determining module 1102 is configured to call the neural network model to determine weights of the conformational ensembles based on the conformational features of the conformational ensembles, where the conformational feature of any conformational ensemble is determined based on the structure data of the conformational ensemble; and call the neural network model to determine the sample prediction result based on the conformational features and the weights of the conformational ensembles.

In a possible implementation, the determining module 1102 is configured to call the neural network model to determine weighted conformational features of the conformational ensembles based on a reference weight and the conformational features of the conformational ensembles; and call the neural network model to perform normalization processing on the weighted conformational features of the conformational ensembles to obtain the weights of the conformational ensembles.

In a possible implementation, the determining module 1102 is configured to call the neural network model to perform weighted calculation based on the conformational features and the weights of the conformational ensembles to obtain a weighted fusion feature; and call the neural network model to determine the sample prediction result based on the weighted fusion feature.

In a possible implementation, the training module 1103 is configured to determine a first loss based on the sample prediction result and the sample labeling data; determine prediction results of the conformational ensembles based on the structure data of the conformational ensembles, where the prediction result of each conformational ensemble is obtained through prediction and is a binding affinity of the conformational ensemble (in other words, the prediction result of each conformational ensemble is obtained through prediction and is configured for representing a binding affinity of the conformational ensemble); for any conformational ensemble, determine a second loss corresponding to the conformational ensemble based on the sample labeling data and the prediction result of the conformational ensemble; and train the neural network model to obtain the binding affinity detection model based on the first loss and second losses corresponding to the conformational ensembles.

In a possible implementation, the training module 1103 is configured to obtain structure data of a benchmark conformational ensemble, where the benchmark conformational ensemble is a real structure formed by the binding of the sample protein and the sample small molecule; determine a labeled category of the conformational ensemble based on the structure data of the benchmark conformational ensemble and the structure data of the target conformational ensemble, where the labeled category of the conformational ensemble is configured for representing whether the conformational ensemble is similar to the benchmark conformational ensemble; and determine the second loss corresponding to the conformational ensemble based on the sample labeling data, and the prediction result and the labeled category of the conformational ensemble.

In a possible implementation, the training module 1103 is configured to determine, in a case that the labeled category of the conformational ensemble is configured for representing that the conformational ensemble is similar to the benchmark conformational ensemble, the second loss corresponding to the conformational ensemble based on a target difference between the prediction result of the conformational ensemble and the sample labeling data; and determine, in a case that the labeled category of the conformational ensemble is configured for representing that the conformational ensemble is not similar to the benchmark conformational ensemble, the second loss corresponding to the conformational ensemble based on a maximum value of the target difference and a set value.

In a possible implementation, the training module 1103 is configured to determine a first loss based on the sample prediction result and the sample labeling data; obtain labeled categories of the candidate conformational ensembles, where the labeled category of each candidate conformational ensemble is configured for representing whether the candidate conformational ensemble is similar to the benchmark conformational ensemble; for any candidate conformational ensemble, determine a third loss corresponding to the candidate conformational ensemble based on the labeled category and the quality indicator of the candidate conformational ensemble; and train the neural network model to obtain the binding affinity detection model based on the first loss and third losses corresponding to the candidate conformational ensembles.

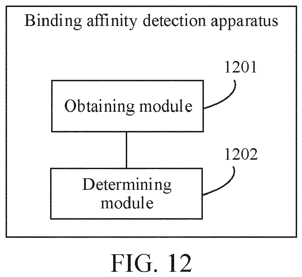

FIG. 12 is a schematic structural diagram of a binding affinity detection apparatus according to an embodiment of the present disclosure. As shown in FIG. 12, the apparatus includes:

an obtaining module 1201, configured to obtain structure data of a target protein, structure data of a target small molecule, and a binding affinity detection model, where the binding affinity detection model is obtained through training based on the method for obtaining a binding affinity detection model according to any one of the foregoing;

a determining module 1202, configured to call the binding affinity detection model to determine structure data of a plurality of reference conformational ensembles based on the structure data of the target protein and the structure data of the target small molecule, where the reference conformational ensemble is a structure obtained through prediction and formed by binding of the target protein and the target small molecule; and

the determining module 1202, further configured to call the binding affinity detection model to determine a target detection result based on the structure data of the reference conformational ensembles, where the target detection result is a detected binding affinity between the target protein and the target small molecule (in other words, the target detection result is obtained through detection and is configured for representing a binding affinity between the target protein and the target small molecule).

In a possible implementation, the determining module 1202 is configured to generate structure data of a plurality of first conformational ensembles based on the structure data of the target protein and the structure data of the target small molecule; call the binding affinity detection model to determine quality indicators of the first conformational ensembles based on the structure data of the first conformational ensembles, where the quality indicator of each first conformational ensemble is configured for indicating quality of the first conformational ensemble; and call the binding affinity detection model to select a plurality of reference conformational ensembles from the plurality of first conformational ensembles based on the quality indicators of the first conformational ensembles, to obtain the structure data of the plurality of reference conformational ensembles.

In a possible implementation, the determining module 1202 is configured to call the binding affinity detection model to determine weights of the reference conformational ensembles based on conformational features of the reference conformational ensembles, where the conformational feature of any reference conformational ensemble is determined based on the structure data of the reference conformational ensemble; and call the binding affinity detection model to determine the target detection result based on the conformational features and the weights of the reference conformational ensembles.

It may be understood that, when the apparatuses provided in FIG. 11 and FIG. 12 implement functions thereof, the foregoing embodiments are merely described by using division of various functional modules as an example. During actual application, the foregoing functions may be allocated to and completed by different functional modules as required. That is, the internal structure of the device is divided into different functional modules, to complete all or some of the functions described above. In addition, the apparatus and method embodiments provided in the foregoing embodiments belong to the same conception. For specific implementation processes and technical effects of the apparatus embodiments, reference may be made to the method embodiments (for example, for a specific implementation process and technical effects of the apparatus provided in FIG. 11, reference may be made to the method embodiment corresponding to FIG. 2, and for a specific implementation process and technical effects of the apparatus provided in FIG. 12, reference may be made to the method embodiment corresponding to FIG. 8), and details are not described herein again.

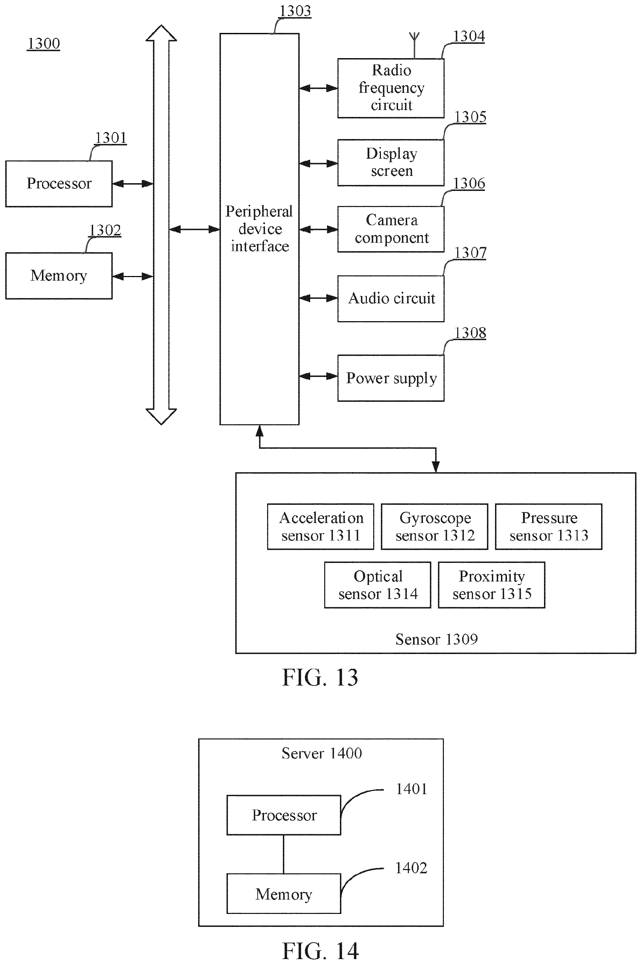

FIG. 13 is a structural block diagram of a terminal device 1300 according to an exemplary embodiment of the present disclosure. The terminal device 1300 includes: a processor 1301 and a memory 1302.

The processor 1301 may include one or more processing cores, such as a 4-core processor or an 8-core processor. The processor 1301 may be implemented by using at least one hardware form of a digital signal processor (DSP), a field-programmable gate array (FPGA), and a programmable logic array (PLA). The processor 1301 may also include a main processor and a coprocessor. The main processor is a processor configured to process data in an active state, also referred to as a central processing unit (CPU). The coprocessor is a low-power consumption processor configured to process data in a standby state. In some embodiments, the processor 1301 may be integrated with a graphics processing unit (GPU). The GPU is configured to render and draw content that needs to be displayed on a display screen. In some embodiments, the processor 1301 may also include an artificial intelligence (AI) processor. The AI processor is configured to process a computing operation related to machine learning.

The memory 1302 may include one or more computer-readable storage media. The computer-readable storage medium may be non-transitory (also referred to as non-temporary). The memory 1302 may also include a high-speed random access memory and a non-volatile memory, for example, one or more disk storage devices and flash storage devices. In some embodiments, the non-transitory computer-readable storage medium in the memory 1302 is configured to store at least one computer program, and the at least one computer program is configured to be executed by the processor 1301 to implement the method for obtaining a binding affinity detection model or the binding affinity detection method provided in the method embodiments of the present disclosure.

In some embodiments, the terminal device 1300 may optionally further include: a peripheral device interface 1303 and at least one peripheral device. The processor 1301, the memory 1302, and the peripheral device interface 1303 may be connected through a bus or a signal cable. Each peripheral device may be connected to the peripheral device interface 1303 through a bus, a signal cable, or a circuit board. Specifically, the peripheral device includes: at least one of a radio frequency circuit 1304, a display screen 1305, a camera component 1306, an audio circuit 1307, and a power supply 1308.

The peripheral device interface 1303 may be configured to connect at least one input/output (I/O)-related peripheral device to the processor 1301 and the memory 1302. In some embodiments, the processor 1301, the memory 1302, and the peripheral device interface 1303 are integrated on the same chip or the same circuit board. In some other embodiments, any one or two of the processor 1301, the memory 1302, and the peripheral device interface 1303 may be implemented on a separate chip or circuit board, which is not limited in this embodiment.

The radio frequency circuit 1304 is configured to receive and transmit a radio frequency (RF) signal that is also referred to as an electromagnetic signal. The RF circuit 1304 communicates with a communication network and another communication device by using the electromagnetic signal. The RF circuit 1304 converts an electric signal into an electromagnetic signal for transmission, or converts a received electromagnetic signal into an electric signal. Optionally, the RF circuit 1304 includes: an antenna system, an RF transceiver, one or more amplifiers, a tuner, an oscillator, a digital signal processor, a codec chipset, a user identity module card, and the like. The RF circuit 1304 may communicate with other terminals through at least one wireless communication protocol. The wireless communication protocol includes but is not limited to: a world wide web, a metropolitan area network, an intranet, generations of mobile communication networks (2G, 3G, 4G, and 5G), a wireless local area network, and/or a wireless fidelity (Wi-Fi) network. In some embodiments, the RF circuit 1304 may also include a circuit related to near field communication (NFC), which is not limited in the present disclosure.

The display screen 1305 is configured to display a user interface (UI). The UI may include a graph, a text, an icon, a video, and any combination thereof. When the display screen 1305 is a touch display screen, the display screen 1305 is further capable of collecting a touch signal on or above a surface of the display screen 1305. The touch signal may be inputted, as a control signal, to the processor 1301 for processing. In this case, the display screen 1305 may also be configured to provide a virtual button and/or a virtual keyboard, also referred to as a soft button and/or a soft keyboard. In some embodiments, there may be one display screen 1305 arranged on a front panel of the terminal device 1300. In some other embodiments, there may be at least two display screens 1305 respectively arranged on different surfaces of the terminal device 1300 or in a folded design. In some other embodiments, the display screen 1305 may be a flexible display screen arranged on a curved or folded surface of the terminal device 1300. Even further, the display screen 1305 may be arranged in a non-rectangular irregular pattern, that is, a special-shaped screen. The display screen 1305 may be prepared by using materials such as a liquid crystal display (LCD), an organic light-emitting diode (OLED), or the like.

The camera component 1306 is configured to collect images or videos. Optionally, the camera component 1306 includes a front-facing camera and a rear-facing camera. Generally, the front-facing camera is arranged on a front panel of the terminal, and the rear-facing camera is arranged on a rear surface of the terminal. In some embodiments, there are at least two rear-facing cameras, each being any one of a main camera, a depth-of-field camera, a wide-angle camera, and a telephoto camera, to achieve a background blurring function through fusion of the main camera and the depth-of-field camera, panoramic photo shooting and virtual reality (VR) shooting functions through fusion of the main camera and the wide-angle camera, or another fusion shooting function. In some embodiments, the camera component 1306 may further include a flash. The flash may be a single color temperature flash or a double color temperature flash. The double color temperature flash refers to a combination of a warm light flash and a cold light flash, and may be used for light compensation under different color temperatures.

The audio circuit 1307 may include a microphone and a speaker. The microphone is configured to collect sound waves from a user and an environment and convert the sound waves into electrical signals that are inputted to the processor 1301 for processing or inputted to the RF circuit 1304 for voice communication. For purposes of stereo collection or noise reduction, there may be a plurality of microphones, which are respectively arranged at different parts of the terminal device 1300. The microphone may be alternatively a microphone array or an omnidirectional acquisition microphone. The speaker is configured to convert the electrical signals from the processor 1301 or the RF circuit 1304 into sound waves. The speaker may be a conventional thin-film speaker or a piezoelectric ceramic speaker. When the speaker is a piezoelectric ceramic speaker, the speaker can not only convert an electric signal into sound waves audible to a human being, but also convert an electric signal into sound waves inaudible to the human being for ranging and other purposes. In some embodiments, the audio circuit 1307 may further include an earphone jack.

The power supply 1308 is configured to supply power to the components in the terminal device 1300. The power supply 1308 may be an alternating-current power supply, a direct-current power supply, a disposable battery, or a rechargeable battery. When the power supply 1308 includes a rechargeable battery, the rechargeable battery may be a wired charging battery or a wireless charging battery. The wired charging battery is a battery charged through a wired line, and the wireless charging battery is a battery charged through a wireless coil. The rechargeable battery may also be configured to support a fast charge technology.

In some embodiments, the terminal device 1300 further includes one or more sensors 1309. The one or more sensors 1309 include but are not limited to: an acceleration sensor 1311, a gyroscope sensor 1312, a pressure sensor 1313, an optical sensor 1314, and a proximity sensor 1315.

The acceleration sensor 1311 may detect accelerations on three coordinate axes of a coordinate system established by the terminal device 1300. For example, the acceleration sensor 1311 may be configured to detect components of a gravitational acceleration on the three coordinate axes. The processor 1301 may control the display screen 1305 to display the user interface in a lateral view or a longitudinal view based on a gravitational acceleration signal collected by the acceleration sensor 1311. The acceleration sensor 1311 may also be configured to collect game or user motion data.

The gyroscope sensor 1312 may detect a body direction and a rotation angle of the terminal device 1300, and the gyroscope sensor 1312 may collect a 3D action performed by a user on the terminal device 1300 in cooperation with the acceleration sensor 1311. The processor 1301 may implement the following functions based on the data collected by the gyroscope sensor 1312: motion sensing (for example, change the UI based on a tilt operation of the user), image stabilization during photographing, game control, and inertial navigation.

The pressure sensor 1313 may be arranged on a side frame of the terminal device 1300 and/or a lower layer of the display screen 1305. When the pressure sensor 1313 is arranged on the side frame of the terminal device 1300, a holding signal of the user to the terminal device 1300 may be detected, and the processor 1301 performs left and right hand recognition or a quick operation based on the holding signal collected by the pressure sensor 1313. When the pressure sensor 1313 is arranged on the lower layer of the display screen 1305, the processor 1301 controls an operable control on the UI based on a pressure operation of the user on the display screen 1305. The operable control includes at least one of a button control, a scroll-bar control, an icon control, and a menu control.

The optical sensor 1314 is configured to collect ambient light intensity. In an embodiment, the processor 1301 may control display brightness of the display screen 1305 based on the ambient light intensity collected by the optical sensor 1314. Specifically, when the ambient light intensity is high, the display brightness of the display screen 1305 is increased; and when the ambient light intensity is low, the display brightness of the display screen 1305 is decreased. In another embodiment, the processor 1301 may also dynamically adjust camera parameters of the camera component 1306 according to the ambient light intensity collected by the optical sensor 1314.

The proximity sensor 1315, also referred to as a distance sensor, is generally arranged on the front panel of the terminal device 1300. The proximity sensor 1315 is configured to collect a distance between the user and a front surface of the terminal device 1300. In an embodiment, when the proximity sensor 1315 detects that the distance between the user and the front surface of the terminal device 1300 is gradually reduced, the processor 1301 controls the display screen 1305 to switch from a screen-on state to a screen-off state. When the proximity sensor 1315 detects that the distance between the user and the front surface of the terminal device 1300 is gradually increased, the processor 1301 controls the display screen 1305 to switch from the screen-off state to the screen-on state.

A person skilled in the art may understand that the structure shown in FIG. 13 constitutes no limitation to the terminal device 1300, and the terminal device may include more or fewer components than those shown in the figure, or some components may be combined, or a different component arrangement may be used.

FIG. 14 is a schematic structural diagram of a server according to an embodiment of the present disclosure. The server 1400 may vary greatly due to different configurations or performance, and may include one or more processors 1401 and one or more memories 1402. The one or more memories 1402 store at least one computer program, and the at least one computer program is loaded and executed by the one or more processors 1401 to implement the method for obtaining a binding affinity detection model or the binding affinity detection method provided in the foregoing method embodiments. For example, the processor 1401 is a CPU. Certainly, the server 1400 may also have components such as a wired or wireless network interface, a keyboard, and an input/output interface to facilitate input/output. The server 1400 may also include other components for implementing device functions. Details are not described herein.

In an exemplary embodiment, a non-transitory computer-readable storage medium is further provided. The non-transitory computer-readable storage medium stores at least one computer program, and the at least one computer program is loaded and executed by a processor to cause an electronic device to implement any one of the foregoing method for obtaining a binding affinity detection model or the foregoing binding affinity detection method.

Optionally, the non-transitory computer-readable storage medium may be a read-only memory (ROM), a random access memory (RAM), a compact disc read-only memory (CD-ROM), a magnetic tape, a floppy disk, an optical data storage device, and the like.

In an exemplary embodiment, a computer program or a computer program product is further provided. The computer program or the computer program product stores at least one computer program, and the at least one computer program is loaded and executed by a processor to cause an electronic device to implement any one of the foregoing method for obtaining a binding affinity detection model or the foregoing binding affinity detection method.

It is to be understood that "a plurality of" mentioned in this specification refers to two or more. "And/or" describes an association relationship between associated objects and represents that three relationships may exist. For example, A and/or B may represent the following three cases: Only A exists, both A and B exist, and only B exists. The character "/" generally represents an "or" relationship between the associated objects.

The sequence numbers of the foregoing embodiments of the present disclosure are merely for description purpose, and do not indicate the preference of the embodiments.

The foregoing descriptions are merely exemplary embodiments of the present disclosure, but are not intended to limit the present disclosure. Any modification, equivalent replacement, or improvement made within the principle of the present disclosure shall fall within the protection scope of the present disclosure.

## Claims

1. A method for obtaining a binding affinity detection model for detecting a binding affinity between a given protein and a given small molecule, executable by an electronic device, and the method comprising:

   obtaining (201) protein structure data of a sample protein, small molecule structure data of a sample small molecule, and a sample labeling data, the sample labeling data representing a binding affinity between the sample protein and the sample small molecule;
   determining (202) structure data of a plurality of conformational ensembles by inputting the protein structure data and the small molecule structure data into an initial predictive neural network model , the plurality of conformational ensembles each indicating a predictive binding structure between the sample protein and the sample small molecule;
   determining (203) a predictive binding affinity between the sample protein and the sample small molecule by inputting the structure data of the conformational ensembles into the initial predictive neural network model; and
   training (204) the initial predictive neural network model using the sample prediction result and the sample labeling data to obtain the binding affinity detection model.

2. The method according to claim 1, wherein the determining structure data of a plurality of conformational ensembles by inputting the protein structure data and the small molecule structure data into an initial predictive neural network model comprises:

   generating structure data of a plurality of candidate conformational ensembles based on the protein structure data and the small molecule structure data;
   determining quality indicators of the candidate conformational ensembles by inoutting the structure data of the candidate conformational ensembles into the initial predictive neural network model, wherein the quality indicator of the candidate conformational ensemble indicates quality of the candidate conformational ensemble; and selecting a plurality of conformational ensembles from the plurality of candidate conformational ensembles by inputting the quality indicators of the candidate conformational ensembles into the initial predictive neural network model, to obtain the structure data of the plurality of conformational ensembles.

3. The method according to claim 2, wherein the protein structure data comprises at least one type, and the generating structure data of a plurality of candidate conformational ensembles based on the protein structure data and the small molecule structure data comprises:

for any type of protein structure data, generating structure data of a candidate conformational ensemble corresponding to the type of protein structure data based on the type of protein structure data and the small molecule structure data; and
determining the structure data of the plurality of candidate conformational ensembles based on structure data of candidate conformational ensembles corresponding to different types of protein structure data.

4. The method according to claim 2 or 3, wherein the determining quality indicators of the candidate conformational ensembles by inputting the structure data of the candidate conformational ensembles into the initial predictive neural network model comprises:

determining a graph structure of the candidate conformational ensemble based on the structure data of the candidate conformational ensemble, wherein the graph structure of the candidate conformational ensemble represents a spatial structure of the candidate conformational ensemble;
determining conformational features of the candidate conformational ensemble by inputting the graph structure of the candidate conformational ensemble into the initial predictive neural network model; and
determining the quality indicators of the candidate conformational ensembles based on the conformational features of the candidate conformational ensembles.

5. The method according to claim 4, wherein the structure data of the candidate conformational ensemble comprises atomic data of a plurality of atoms, each of the plurality of atoms is an atom in the sample protein or an atom in the sample small molecule, the graph structure of the candidate conformational ensemble comprising a plurality of nodes and a plurality of edges;
wherein the determining a graph structure of the candidate conformational ensemble based on the structure data of the candidate conformational ensemble comprises:

for each of the candidate conformational ensembles, determining a distance between every two atoms based on the atomic data of the plurality of atoms comprised in the candidate conformational ensemble;
determining the nodes comprised in the graph structure of the candidate conformational ensemble based on the atomic data of the atoms comprised in the candidate conformational ensemble; and
for any two nodes comprised in the graph structure of the candidate conformational ensemble, adding an edge between the two nodes in a case that a distance between two atoms corresponding to the two nodes is less than a distance threshold.

6. The method according to any one of claims 1 to 5, wherein the determining a sample prediction result by inputting the structure data of the conformational ensembles into the initial predictive neural network model comprises:

determining weights of the target conformational ensembles by inputting conformational features of the conformational ensembles into the initial predictive neural network model, wherein the conformational feature of the conformational ensemble is determined based on the structure data of the target conformational ensemble; and
determining the sample prediction result by inputting the conformational features and the weights of the conformational ensembles into the initial predictive neural network model.

7. The method according to claim 6, wherein the determining weights of the conformational ensembles by inputting conformational features of the conformational ensembles into the initial predictive neural network model comprises:

determining weighted conformational features of the conformational ensembles by inputting the conformational features of the conformational ensembles and a reference weight into the initial predictive neural network model; and

performing normalization processing on the weighted conformational features of the conformational ensembles, to obtain the weights of the conformational ensembles.

8. The method according to claim 6 or 7, wherein the determining the sample prediction result by inputting the conformational features and the weights of the conformational ensembles into the initial predictive neural network model comprises:

performing weighted calculation by inputting the conformational features and the weights of the conformational ensembles into the initial predictive neural network model to obtain a weighted fusion feature; and
determining the sample prediction result by inputting the weighted fusion feature into the initial predictive neural network model.

9. The method according to any one of claims 1 to 8, wherein the training the neural network model by using the sample prediction result and the sample labeling data to obtain a binding affinity detection model comprises:

determining a first loss based on the sample prediction result and the sample labeling data;
determining prediction results of the conformational ensembles based on the structure data of the conformational ensembles, wherein the prediction result of the target conformational ensemble is obtained through prediction and represents a binding affinity of the target conformational ensemble;
for each of the conformational ensembles, determining a second loss of the conformational ensemble based on the sample labeling data and the prediction result of the conformational ensemble; and
training the initial predictive neural network model to obtain the binding affinity detection model based on second losses of the conformational ensembles and the first loss.

10. The method according to claim 9, wherein the determining a second loss of the conformational ensemble based on the sample labeling data and the prediction result of the conformational ensemble comprises:

obtaining structure data of a benchmark conformational ensemble, wherein the benchmark conformational ensemble is a real structure formed by the binding the sample protein and the sample small molecule;
determining a labeling category of the conformational ensemble based on the structure data of the benchmark conformational ensemble and the structure data of the conformational ensemble, wherein the labeling category of the conformational ensemble represents whether the conformational ensemble is similar to the benchmark conformational ensemble; and
determining the second loss of the conformational ensemble based on the sample labeling data, and the prediction result and the labeling category of the conformational ensemble.

11. The method according to claim 10, wherein the determining the second loss of the conformational ensemble based on the sample labeling data, and the prediction result and the labeling category of the conformational ensemble comprises:

in a case that the labeling category of the conformational ensemble represents that the conformational ensemble is similar to the benchmark conformational ensemble, determining the second loss of the conformational ensemble based on a target difference between the prediction result of the conformational ensemble and the sample labeling data; and
in a case that the labeling category of the conformational ensemble represents that the conformational ensemble is not similar to the benchmark conformational ensemble, determining the second loss of the conformational ensemble based on a maximum value between the target difference and a set value.

12. The method according to any one of claims 1 to 8, wherein the training the neural network model by using the sample prediction result and the sample labeling data to obtain a binding affinity detection model comprises:

determining a first loss based on the sample prediction result and the sample labeling data;
obtaining labeling categories of the candidate conformational ensembles, wherein the labeling category of each of the candidate conformational ensembles represents whether the candidate conformational ensemble is similar to a benchmark conformational ensemble;
for each of the candidate conformational ensembles, determining a third loss of the candidate conformational ensemble based on the labeling category and the quality indicator of the candidate conformational ensemble; and
training the neural network model to obtain the binding affinity detection model based on third losses of the candidate conformational ensembles and the first loss.

13. A binding affinity detection method, executable by an electronic device, and the method comprising:

obtaining (801) structure data of a given protein, structure data of a given small molecule, and a binding affinity detection model, the binding affinity detection model being obtained through training by using the method for obtaining a binding affinity detection model according to any one of claims 1 to 12;

determining (802) structure data of a plurality of reference conformational ensembles by inputting the structure data of the given protein and the structure data of the given small molecule into the binding affinity detection model, the plurality of reference conformational ensembles each indicating a structure obtained through prediction and formed by binding the given protein and the given small molecule; and

determining (803) a binding affinity detection result between the given protein and the given small molecule by inputting the structure data of the reference conformational ensembles into the binding affinity detection model.

14. The method according to claim 13, wherein the determining structure data of a plurality of reference conformational ensembles by inputting the structure data of the given protein and the structure data of the given small molecule comprises:

generating structure data of a plurality of first conformational ensembles based on the structure data of the target protein and the structure data of the given small molecule;

determining quality indicators of the first conformational ensembles by inputting the structure data of the first conformational ensembles into the binding affinity detection model, wherein the quality indicator of each of the plurality of first conformational ensembles indicates quality of the first conformational ensemble; and

selecting a plurality of reference conformational ensembles from the plurality of first conformational ensembles by inputting the quality indicators of the first conformational ensembles into the binding affinity detection model, to obtain the structure data of the plurality of reference conformational ensembles.

15. The method according to claim 13 or 14, wherein the determining a binding affinity detection result between the given protein and the given small molecule by inputting the structure data of the reference conformational ensembles into the binding affinity detection model comprises:

determining weights of the reference conformational ensembles by inputting conformational features of the reference conformational ensembles into the binding affinity detection model, wherein the conformational feature of each of the reference conformational ensembles is determined based on the structure data of the reference conformational ensemble; and

determining the binding affinity detection result by inputting the conformational features and the weights of the reference conformational ensembles into the binding affinity detection model.

16. An apparatus for training a binding affinity detection model, comprising:

an obtaining module, configured to obtain protein structure data of a sample protein, small molecule structure data of a sample small drug molecule, and a sample labeling result, the sample labeling result being obtained through labeling and being configured for representing a binding affinity between the sample protein and the sample small drug molecule;

a determining module, configured to call a neural network model to determine structure data of a plurality of target complex conformations based on the protein structure data and the small molecule structure data, any target complex conformation being a structure obtained through prediction and formed by binding of the sample protein and the sample small drug molecule;

the determining module, further configured to call the neural network model to determine a sample prediction result based on the structure data of the target complex conformations, the sample prediction result being obtained through prediction and being configured for representing the binding affinity between the sample protein and the sample small drug molecule; and

a training module, configured to train the neural network model by using the sample prediction result and the sample labeling result to obtain a binding affinity detection model, the binding affinity detection model being configured for detecting a binding affinity between a target protein and a target small drug molecule.

17. A binding affinity detection apparatus, comprising:

an obtaining module, configured to obtain structure data of a target protein, structure data of a target small drug molecule, and a binding affinity detection model, the binding affinity detection model being obtained through training based on the method for training a binding affinity detection model according to any one of claims 1 to 12; a determining module, configured to call the binding affinity detection model to determine structure data of a plurality of reference complex conformations based on the structure data of the target protein and the structure data of the target small drug molecule, the reference complex conformation being a structure obtained through prediction and formed by binding of the target protein and the target small drug molecule; and the determining module, further configured to call the binding affinity detection model to determine a target detection result based on the structure data of the reference complex conformations, the target detection result being obtained through detection and being configured for representing a binding affinity between the target protein and the target small drug molecule.

18. An electronic device, comprising a processor and a memory, the memory storing at least one computer program, the at least one computer program being loaded and executed by the processor to cause the electronic device to implement the method for training a binding affinity detection model according to any one of claims 1 to 12, or implement the binding affinity detection method according to any one of claims 13 to 15.

19. A non-transitory computer-readable storage medium, storing at least one computer program, the at least one computer program being loaded and executed by a processor to cause an electronic device to implement the method for training a binding affinity detection model according to any one of claims 1 to 12, or implement the binding affinity detection method according to any one of claims 13 to 15.

20. A computer program product, comprising at least one computer program, the at least one computer program being loaded and executed by a processor to cause an electronic device to implement the method for training a binding affinity detection model according to any one of claims 1 to 12, or implement the binding affinity detection method according to any one of claims 13 to 15.

FIG. 1

| |
|---|
| Obtain protein structure data of a sample protein, small molecule structure data of a sample small molecule, and a sample labeling data |

201

| |
|---|
| Determine structure data of a plurality of conformational ensembles by inputting the protein structure data and the small molecule structure data into an initial predictive neural network model |

202

| |
|---|
| Determine a predictive binding affinity between the sample protein and the sample small molecule by inputting the structure data of the conformational ensembles into the initial predictive neural network model |

203

| |
|---|
| Train the initial predictive neural network model using the sample prediction result and the sample labeling data to obtain the binding affinity detection model |

204

FIG. 2

Spatial structure of
candidate conformational
ensemble

Atom of sample
small molecule

Atom of
sample protein

Graph structure of
candidate conformational
ensemble

FIG. 3

Graph structure of
candidate conformational
ensemble

Atom in sample
small molecule

Atom in sample protein

P1   P2   P3

Atomic feature
of sample small
molecule

Feature of
sample small
molecule

⊕

Atomic feature
of sample
protein

Feature
of sample
protein

L1

Feature of
each edge

Edge
feature

e11  e21  e31

Conformational
feature of
candidate
conformational
ensemble

FIG. 4

FIG. 5

FIG. 6

FIG. 7

801

Obtain structure data of a given protein, structure data of a given small molecule, and a binding affinity detection model

802

Determine structure data of a plurality of reference conformational ensembles by inputting the structure data of the given protein and the structure data of the given small molecule into the binding affinity detection model

803

Determine a binding affinity detection result between the given protein and the given small molecule by inputting the structure data of the reference conformational ensembles into the binding affinity detection model

FIG. 8

FIG. 9

FIG. 10

FIG. 11

Binding affinity detection apparatus

Obtaining module       1201

Determining module       1202

FIG. 12

1303

1300

1301

Processor

1302

Memory

Peripheral device interface

1304
Radio frequency circuit

1305
Display screen

1306
Camera component

1307
Audio circuit

1308
Power supply

Acceleration sensor 1311

Gyroscope sensor 1312

Pressure sensor 1313

Optical sensor 1314

Proximity sensor 1315

Sensor 1309

FIG. 13

Server 1400

Processor — 1401

Memory — 1402

FIG. 14

## INTERNATIONAL SEARCH REPORT

| International application No. |
|---|
| **PCT/CN2023/113188** |

**A. CLASSIFICATION OF SUBJECT MATTER**

G06N3/04(2023.01)i

According to International Patent Classification (IPC) or to both national classification and IPC

**B. FIELDS SEARCHED**

Minimum documentation searched (classification system followed by classification symbols)

IPC:G06N,G16B,G06Q

Documentation searched other than minimum documentation to the extent that such documents are included in the fields searched

Electronic data base consulted during the international search (name of data base and, where practicable, search terms used)

CNTXT, ENTXTC, VEN, IEEE, CNKI: 亲和力, 蛋白质, 小分子, 药物, 预测, 检测, 模型, 神经网络, 图, 标注, 标识, 损失, affinity, small molecule, compound, protein, feature, graph, loss, Neural network

**C. DOCUMENTS CONSIDERED TO BE RELEVANT**

| Category* | Citation of document, with indication, where appropriate, of the relevant passages | Relevant to claim No. |
|---|---|---|
| X | CN 114333986 A (TENCENT TECHNOLOGY (SHENZHEN) CO., LTD.) 12 April 2022 (2022-04-12) description, paragraphs 0060-0194, and figures 2-9 | 1-3, 6-20 |
| Y | CN 114333986 A (TENCENT TECHNOLOGY (SHENZHEN) CO., LTD.) 12 April 2022 (2022-04-12) description, paragraphs 0060-0194, and figures 2-9 | 4-5 |
| Y | CN 114386694 A (PING AN TECHNOLOGY (SHENZHEN) CO., LTD.) 22 April 2022 (2022-04-22) description, paragraphs 0025-0082 | 4-5 |
| A | CN 113744799 A (CENTRAL SOUTH UNIVERSITY) 03 December 2021 (2021-12-03) entire document | 1-20 |
| A | CN 112289371 A (BEIJING STONEWISE TECHNOLOGY CO., LTD.) 29 January 2021 (2021-01-29) entire document | 1-20 |
| A | CN 114999565 A (DALIAN MARITIME UNIVERSITY) 02 September 2022 (2022-09-02) entire document | 1-20 |

☑ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **29 October 2023** | **02 November 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/113188** |

**C. DOCUMENTS CONSIDERED TO BE RELEVANT**

| Category* | Citation of document, with indication, where appropriate, of the relevant passages | Relevant to claim No. |
|---|---|---|
| A | KR 20220010327 A (ARONTIER CO., LTD.) 25 January 2022 (2022-01-25) entire document | 1-20 |

Form PCT/ISA/210 (second sheet) (July 2022)

**INTERNATIONAL SEARCH REPORT**
**Information on patent family members**

International application No.

**PCT/CN2023/113188**

| Patent document cited in search report | | | Publication date (day/month/year) | Patent family member(s) | | | Publication date (day/month/year) |
|---|---|---|---|---|---|---|---|
| CN | 114333986 | A | 12 April 2022 | None | | | |
| CN | 114386694 | A | 22 April 2022 | None | | | |
| CN | 113744799 | A | 03 December 2021 | None | | | |
| CN | 112289371 | A | 29 January 2021 | None | | | |
| CN | 114999565 | A | 02 September 2022 | None | | | |
| KR | 20220010327 | A | 25 January 2022 | KR | 102576033 | B1 | 11 September 2023 |

**EP 4 401 008 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211168243